# EUROPEAN PATENT APPLICATION

(11) **EP 1 579 864 A1**
(43) Date of publication of application: **28.09.2005**
(21) Application number: 05104506.0
(22) Date of filing: 23.04.2003
(51) Int. Cl.: A61K 31/7048, C07H 17/08

(54) **Oxolide antibacterials**

(30) Priority: 25.04.2002 US 132121; 22.04.2003 US 420257
(62) Divisional of application: 03719889.2
(71) Applicant: ABBOTT LABORATORIES, Illinois 60064-6008 (US)
(72) Inventor: MA, Zhenkun, 75225-4627, Dallas, TX (US); DJURIC, Stevan, 60048, Libertyville,Illinois (US); FLORJANCIC, Alan S., 53144, Kenosha, WI (US); YONG, Hong, 60030, Grayslake, IL (US); GU, Yugui, 60048, Libertyville, IL (US)
(74) Representative: Modiano, Guido

(57) **Abstract**

Antibacterial compounds having formula (II), and salts, prodrugs, and salts of prodrugs thereof, processes for making the compounds and intermediates employed in the processes, compositions containing the compounds, and methods for prophylaxis or treatment of bacterial infections in a fish or a mammal using the compounds are disclosed.

## Description

### TECHNICAL FIELD

This invention is directed to compounds which are useful as antibacterials, processes for making the compounds and intermediates useful in the process, compositions containing the compounds, and methods for prophylaxis or treatment of bacterial infections using the compounds.

### BACKGROUND OF THE INVENTION

Because the effectiveness of many drugs currently available for prophylaxis or treatment of bacterial infections is being compromised by the emergence of drug-resistant bacteria, novel antibacterials would be beneficial for their therapeutic value and their contribution to the antibacterial arts.

### SUMMARY OF THE INVENTION

A first embodiment of this invention, therefore, is directed to compounds which are useful as antibacterials, and salts, prodrugs, and salts of prodrugs thereof, the compounds having formula (I) 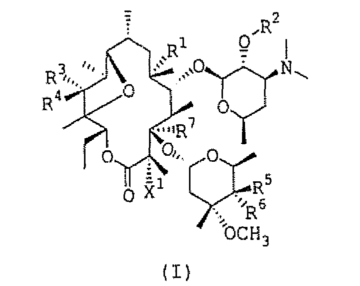 or formula (II) 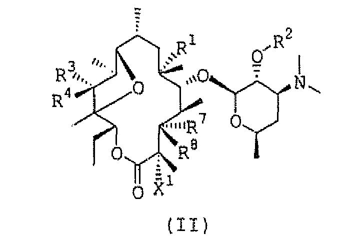 in which
R¹ is hydrogen, -OH, -OR⁹, -OC(O)OR⁹, -OC(O)NH₂, -OC(O)NHR¹⁰, -OC(O)NR¹⁰R¹¹, -OCH₂R¹², -OC(O)OCH₂R¹², -OC(O)NHCH₂R¹², or -OC(O)N(CH₂R¹²)₂;
R² is hydrogen or R^{p}, in which R^{p} is a hydroxyl protecting moiety;
one of R³ and R⁴ is hydrogen, and the other is -OH, -OR¹³, -OC(O)OR¹³, -NH₂, -NHC(O)OR¹⁴, -NHR¹⁵, -NR¹⁵R¹⁶, -OC(O)NH₂, -OC(O)NHR¹⁵, -OC(O)NR¹⁵R¹⁶, -N(R¹⁷)C(O)NH₂, -N(R¹⁷)C(O)NHR¹⁵, -N(R¹⁷)C(O)NR¹⁵R¹⁶, -OCH₂R¹⁸, -NHCH₂R¹⁸, -N(CH₂R¹⁸)₂, -OC(O)OCH₂R¹⁸, -OC(O)NHCH₂R¹⁸, -OC(O)N(CH₂R¹⁸)₂, -N(R¹⁷)C(O)NHCH₂R¹⁸, or -N(R¹⁷)C(O)N(CH₂R¹⁸)₂; or
R³ and R⁴ together are =O or =NOR¹⁹;
one of R⁵ and R⁶ is hydrogen, and the other is -OH, -OR²⁰, -OC(O)OR²⁰, -NH₂, -NHC(O)OR¹⁴, -NHR²¹, -NR²¹R²², -OC(O)NH₂, -OC(O)NHR²¹, -OC(O)NR²¹R²², -N(R²³)C(O)NH₂, -N(R²³)C(O)NHR²¹, -N(R²³)C(O)NR²¹R²², -OCH₂R²⁴, -NHCH₂R²⁴, -N(CH₂R²⁴)₂, -OC(O)OCH₂R²⁴, -OC(O)NHCH₂R²⁴, -OC(O)N(CH₂R²⁴)₂, -N(R²³)C(O)NHCH₂R²⁴, or -N(R²³)C(O)N(CH₂R²⁴)₂; or
R⁵ and R⁶ together are =O;
R⁷ is hydrogen and R⁸ is -OH, -OR²⁵, -OC(O)R²⁵, -OC(O)OR²⁵, -OC(O)NH₂, -OC(O)NHR²⁶, -OC(O)NR²⁶R²⁷, -OCH₂R²⁸, or -OC(O)OCH₂R²⁸; or
R⁷ and R⁸ together are =O;
R⁹, R¹³, R¹⁹, R²⁰, and R²⁵ are independently alkyl, cycloalkyl, -(CH₂)alkenyl, -(CH₂)alkynyl, alkyl substituted with one substituent selected from the group consisting of cycloalkyl, aryl, heteroaryl, and heterocyclyl, -(CH₂)alkenyl substituted with one substituent selected from the group consisting of cycloalkyl, aryl, heteroaryl, and heterocyclyl, or -(CH₂)alkynyl substituted with one substituent selected from the group consisting of cycloalkyl, aryl, heteroaryl, and heterocyclyl;
R¹⁰, R¹¹, R¹⁵, R¹⁶, R²¹, R²², R²⁶, and R²⁷ are independently alkyl, cycloalkyl, -(CH₂)alkenyl, -(CH₂)alkynyl, aryl, heteroaryl, heterocyclyl, alkyl substituted with one substituent selected from the group consisting of cycloalkyl, aryl, heteroaryl, heterocyclyl, -NH₂, -NHR³¹, and -NR³¹R³², -(CH₂)alkenyl substituted with one substituent selected from the group consisting of cycloalkyl, aryl, heteroaryl, heterocyclyl, -NH₂, -NHR³¹, and -NR³¹R³², or -(CH₂)alkynyl substituted with one substituent selected from the group consisting of cycloalkyl, aryl, heteroaryl, heterocyclyl, -NH₂, -NHR³¹, and -NR³¹R³²; or
R¹⁰ and R¹¹ together, R¹⁵ and R¹⁶ together, R²¹ and R²² together, or R²⁶ and R²⁷ together are independently C₃-C₆-alkylene, C₅-C₆-alkylene interrupted with one moiety selected from the group consisting of -O-, -NH-, -N(alkyl)-, -S-, -S(O)-, and -SO₂-, C₃-C₆-alkylene substituted with one substituent selected from the group consisting of -OH, -O(alkyl), =O, -NH₂, -NH(alkyl), and -N(alkyl)₂, or C₅-C₆-alkylene interrupted with one moiety selected from the group consisting of -O-, -NH-, -N(alkyl)-, -S-, -S(O)-, and -SO₂- and substituted with one substituent selected from the group consisting of -OH, -O(alkyl) , =O, -NH₂, -NH(alkyl), and -N(alkyl)₂;
R¹², R¹⁸, R²⁴, and R²⁸ are independently alkyl interrupted with one, two, or three moieties independently selected from the group consisting of -O-, -NH-, -N(alkyl)-, -S-, -S(O)-, and -SO₂- or alkyl interrupted with one, two, or three moieties independently selected from the group consisting of -O-, -NH-, -N(alkyl)-, -S-, -S(O)-, and -SO₂-and substituted with one, two, or three substituents independently selected from the group consisting of cycloalkyl, halo, aryl, heteroaryl, heterocyclyl -OH, =O, -O(alkyl), -NH₂, -NH(alkyl), and -N(alkyl)₂;
R¹⁴ is alkyl or alkyl substituted with one or two independently selected aryl substituents;
R¹⁷ and R²³ are independently hydrogen or alkyl;
R³¹ and R³² are independently alkyl, cycloalkyl, aryl, heteroaryl, heterocyclyl, -(CH₂)alkenyl, -(CH₂)alkynyl, alkyl substituted with one substituent selected from the group consisting of cycloalkyl, aryl, heteroaryl, heterocyclyl, -NH₂, -NH(alkyl), and -N(alkyl)₂, -(CH₂)alkenyl substituted with one substituent selected from the group consisting of cycloalkyl, aryl, heteroaryl, heterocyclyl, -NH₂, -NH(alkyl), and -N(alkyl)₂, or -(CH₂)alkynyl substituted with one substituent selected from the group consisting of cycloalkyl, aryl, heteroaryl, heterocyclyl, -NH₂, -NH(alkyl), and -N(alkyl)₂; or
R³¹ and R³² together are C₃-C₆-alkylene, C₅-C₆-alkylene interrupted with one moiety selected from the group consisting of -O-, -NH-, -N(alkyl)-, -S-, -S(O)-, and -SO₂-, C₃-C₆-alkylene substituted with one substituent selected from the group consisting of -OH, -O(alkyl), =O, -NH₂, -NH(alkyl), and -N(alkyl)₂, or C₅-C₆-alkylene interrupted with one moiety selected from the group consisting of -O-, -NH-, -N(alkyl)-, -S-, -S(O)-, and -SO₂- and substituted with one substituent selected from the group consisting of -OH, -C(alkyl), =O, -NH₂, -NH(alkyl), and -N(alkyl)₂; and
X¹ is hydrogen, fluoride, chloride, or bromide.

A second embodiment of this invention is directed to the compounds of the first embodiment, and the salts, prodrugs, and salts of the prodrugs thereof, having the stereochemistry shown in the compounds having formula (I)-f, 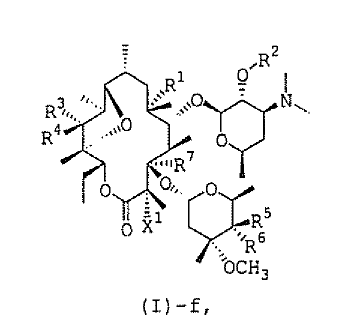 formula (I)-g, 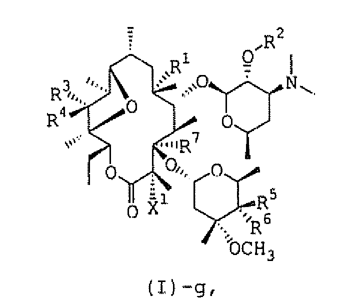 formula (II)-f, 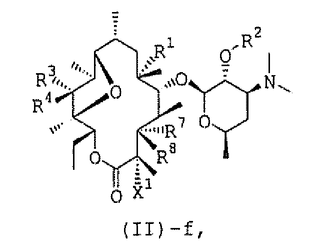 or formula (II)-g,

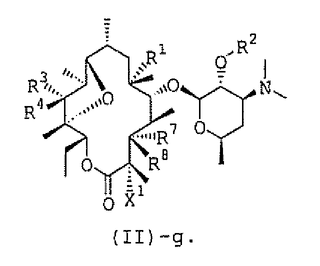

A third embodiment of this invention is directed to a process for making the compounds of the first and second embodiments.

A fourth embodiment of this invention is directed to intermediates which are useful in the second embodiment.

A fifth embodiment of this invention is directed to compositions for the prophylaxis or treatment of bacterial infections in a fish or a mammal, the compositions comprising a therapeutically effective amount of one or more of the compounds of the first or second embodiment and an excipient.

A sixth embodiment of this invention is directed to use of a therapeutically effective amount of a compound of the first or second embodiment for preparation of a medicament for prophylaxis or treatment of bacterial infections.

### DETAILED DESCRIPTION OF THE INVENTION

Compounds of this invention, also referred to as "the compounds," comprise of both fixed and variable moieties, which variable moieties are identified by a capital letter and accompanying numerical or alphabetical superscript, and for which the following terms have the meanings indicated.

"Alkenyl" means monovalent, straight-chain and branched-chain hydrocarbon moieties, having two to eight carbon atoms and at least one carbon-carbon double bond. Alkenyl moieties include but-1,3-dienyl, butenyl, but-2-enyl, ethenyl, 1-ethylhexen-2-yl, hex-3-enyl, 1-methylbutenyl, 2-methylbutenyl, 1-methylbut-2-enyl, 1-methylbut-1,3-dienyl, pentenyl, pent-2-enyl, pent-3-enyl, and propenyl.

"Alkyl" means monovalent, saturated, straight-chain and branched-chain hydrocarbon moieties, having one to six carbon atoms. Alkyl moieties include butyl, 1,1,-dimethylethyl (tert-butyl), 1,1-dimethylpropyl, 1,2-dimethylpropyl, ethyl, 1-ethylpropyl, 2-ethylpropyl, hexyl, methyl, 2-methylpropyl, 3-methylbutyl, 1-methylpentyl, 2-methylpent-3-yl, and pentyl.

"Alkylene" means divalent, saturated, straight-chain and branched-chain hydrocarbon moieties, having one to eight carbon atoms. Alkylene moieties include butylene, 1,1,-dimethylethylene, 1,1-dimethylpropylene, 1,2-dimethylpropylene, ethylene, 1-ethylpropylene, 2-ethylpropylene, hexylene, methylene, 2-methylpropylene, 3-methylbutylene, 1-methylpentylene, 2-methylpent-3-ylene, and pentylene.

"Alkynyl" means monovalent, straight-chain and branched-chain hydrocarbon moieties, having two to six carbon atoms and at least one carbon-carbon triple bond. Alkynyl moieties include ethynyl (acetylenyl), pentynyl, pent-2-ynyl, pent-3-ynyl, pent-4-ynyl, 1-methylbut-2-ynyl, 2-methylbut-3-ynyl, hexynyl, hex-2-ynyl, hex-3-ynyl, hex-4-ynyl, 1-methyl-pent-2-ynyl, 1-methylenepent-3-ynyl, 1-methyl-pent-2,4-diynyl, and prop-2-ynyl (propargyl).

"Aryl" means monovalent, unsubstituted or substituted phenyl which is unfused or fused with another phenyl moiety or a cycloalkyl, cycloalkenyl, heteroaryl, heterocyclyl, naphthyl, or saturated part of an indanyl moiety.

Phenyl moieties fused with phenyl, naphthyl, or the saturated part of an indanyl moieties are unsubstituted and substituted naphthyl, anthracen-(1- to 4-)yl, or fluoren-(1-to 4-)yl, respectively.

Phenyl moieties fused with cycloalkyl moieties are unsubstituted and substituted indan-(4- to 7-)yl and 1,2,3,4-tetrahydronaphth-(5- to 8-)yl,

Phenyl moieties fused with cycloalkenyl moieties are unsubstituted and substituted inden-(4- to 7-)yl, 1,2-dihydronaphth-(5- to 8-)yl and 1,2-dihydronaphth-(5- to 8-)yl.

Phenyl moieties fused with heteroaryl moieties include unsubstituted and substituted benzimidazol-(4- to 7-)yl, 1-benzofuran-(4- to 7-)yl, 1,2-benzisothiazol-(4- to 7-)yl, benzthiazol-(4- to 7-)yl, 1-benzothiophen-(4- to 7-)yl, cinnolin-(5- to 8-)yl, indol-(4- to 7-)yl, isoquinolin-(5-to 8-)yl, phthalazin-(5- to 8-)yl, quinazolin-(5- to 8-)yl, quinolin-(5- to 8-)yl, and quinoxalin-(5- to 8-)yl.

Phenyl moieties fused with heterocyclyl moieties include unsubstituted and substituted 1,3-benzodioxa(4- to 7-)yl, 1,4-benzodioxa(5- to 8-)yl, 1,3-dihydro-2-benzofuran-(4- to 7-)yl, 2,3-dihydro-1-benzofuran-(4- to 7-)yl, 1,3-dihydro-2-benzothiophen-(4- to 7-)yl, 2,3-dihydro-1-benzothiophen-(4- to 7-)yl, and indolin-(4- to 7-)yl.

"Cycloalkyl" means monovalent, unsubstituted and substituted, saturated cyclic hydrocarbon moieties, having three to six carbon atoms. Cycloalkyl moieties are unsubstituted and substituted cyclopropyl, cyclobutyl, cyclopentyl, and cyclohexyl.

"Cycloalkenyl" means means monovalent, unsubstituted and substituted, cyclic hydrocarbon moieties having four to six carbon atoms and at least one carbon-carbon double bond. Cycloalkenyl moieties are unsubstituted and substituted 1,3-cyclohexadienyl, 1,4-cyclohexadienyl, cyclohexenyl, cyclopentadienyl, and cyclopentenyl.

"Halo" means fluoro (-F), chloro (-Cl), bromo (-Br), and iodo (-I).

"Heteroaryl" means monovalent, aromatic, unsubstituted and substituted five-membered ring moieties having two double bonds and (a) one oxygen or one sulfur atom, (b) one, two, three, or four nitrogen atoms, or (c) one or two nitrogen atoms and one oxygen or one sulfur atom and the remaining atoms are carbon atoms, each of which is attached through a carbon atom or a nitrogen atom; and monovalent six-membered ring moieties having three double bonds and one, two, or three nitrogen atoms and the remaining atoms are carbon atoms, attached through a carbon atom; in which the foregoing heteroaryl moieties are unfused or fused with another heteroaryl moiety or an aryl moiety.

Five-membered heteroaryl moieties are unsubstituted and substituted furanyl, imidazolyl, isothiazolyl, isoxazolyl, 1,2,3-oxadiazolyl, oxazolyl, pyrazolyl, pyrrolyl, tetrazolyl, 1,3,4-thiadiazolyl, thiazolyl, thiophenyl (thienyl), 2H-tetraäzolyl, and 1,2,3-triazolyl,

Five-membered heteroaryl moieties fused with aryl moieties include unsubstituted and substituted benzimidazol-(1- or 2-)yl, 1-benzofuran-(2- to 3-)yl, 1,2-benzisothiazol-3-yl, benzthiazol-2-yl, 1-benzothiophen-(2- to 3-)yl, cinnolin-(3- or 4-)yl, indol-(1-to 3-)yl, isoquinolin-(1-, 3-, or 4-)yl, phthalazin-(1- or 4-)yl, quinazolin-(2- or 4-)yl, quinolin-(2- to 4-)yl, and quinoxalin-(2- or 3-)yl.

Five-membered heteroaryl moieties fused with other five-membered heteroaryl moieties include unsubstituted and substituted [1,3]thiazolo[4,5-d][1,3]oxazolyl, [1,3]thiazolo[4,5-d][1,3]thiazolyl, thieno[3,2-d][1,3]oxazolyl, thieno[3,2-d][1,3]thiazolyl, and thieno[2,3-b]thiophenyl.

Five-membered heteroaryl moieties fused with six-membered heteroaryl moieties include unsubstituted and substituted furo [2,3-b] pyridin-(2- or 3-)yl, 3H-imidazo [4,5-b] pyridin-(2- or 3-)yl, [1,3]thiazolo [4,5-b] pyrazin-2-yl, [1,3] thiazolo [4,5-b] pyridin-2-yl, and thieno[2,3-b]pyridin-(2- or 3-)yl.

Six-membered heteroaryl moieties are unsubstituted and substituted pyrazinyl, pyridazinyl, pyridyl, pyrimidinyl, and 1,3,5-triazinyl.

Six-membered heteroaryl moieties fused with aryl moieties include unsubstituted and substituted cinnolin-(3-or 4-) yl, isoquinolin-(1-, 3-, or 4-) yl, phthalazin-(1- or 4-) yl, quinazolin-(2- or 4-) yl, quinolin-(2- to 4-) yl, and quinoxalin-(2- or 3-) yl.

Six-membered heteroaryl moieties fused with five-membered heteroaryl moieties include unsubstituted and substituted furo [2,3-b] pyridin-(4- to 6-) yl, 3H-imidazo [4,5-b] pyridin-(5- to 7-)yl, [1,3] thiazolo [4,5-b] pyrazin-(5- or 6-)yl, [1,3] thiazolo [4,5-b] pyridin-(5- to 7-)yl, and thieno [2,3-b] pyridin-(4- to 6-)yl.

Six-membered heteroaryl moieties fused with other six-membered heteroaryl moieties include unsubstituted and substituted 1,5-naphthyridinyl, 1,7-naphthyridinyl, 1,8-naphthyridinyl, pteridinyl, pyridazino [4,5-d] pyridazinyl, pyrido [2,3-d] pyridazinyl, and pyrido [3,4-d] pyridazinyl.

"Heterocyclyl" means (a) monovalent, non-aromatic, unsubstituted and substituted four-membered ring moieties having one nitrogen, oxygen, or sulfur atom and the remaining atoms are carbon atoms, zero double bonds, attached through a carbon atom or a nitrogen atom, (b) monovalent, non-aromatic, unsubstituted and substituted five-membered ring moieties having one or two nitrogen, oxygen, or sulfur atoms and the remaining atoms are carbon atoms, and zero or one double bonds, attached through a carbon atom or a nitrogen atom, and (c) monovalent, non-aromatic, unsubstituted and substituted six-membered ring moieties having one, two, or three nitrogen, oxygen, or sulfur atoms and the remaining atoms are carbon atoms, and zero, one, or two double bonds, attached through a carbon atom or a nitrogen atom.

Four-membered heterocyclyl moieties are unsubstituted and substituted oxetane, thietane, and azetidine.

Five-membered heterocyclyl moieties include unsubstituted and substituted 1,4-dioxanyl, 1,3-dioxolanyl, imidazolidinyl, 2-imidazolinyl, 4,5-dihydroisoxazolyl, pyrazolidinyl, 2-pyrazolinyl, pyrrolidinyl, 2-pyrrolinyl, 3-pyrrolinyl, and 2H-pyrrolyl,

Six-membered heterocyclyl moieties include unsubstituted and substituted 1,3-dithianyl, 1,4-dithianyl, morpholinyl, piperidinyl, piperazinyl, pyranyl, 2H-pyranyl, 4H-pyranyl, and thiomorpholinyl.

Substituted aryl and heteroaryl moieties are those moieties substituted with one, two, three, four, or five substituents independently selected from the group consisting of alkyl, alkenyl, alkynyl, cycloalkyl, halo, -CN, -OH, -SH, -NH₂, -NO₂, -CF₃, -CH₂CF₃, -CF₂CF₃, -OCF₃, -OCH₂CF₃, -OCF₂CF₃, -OR³⁰, -SR³⁰, -S(O)(alkyl), -SO₂(alkyl), -C(O)H, -C(O)(alkyl), -C(O)OH, -C(O)O(alkyl), -NH(alkyl), -N(alkyl)₂, -C(O)NH₂, -C(O)NH(alkyl), -C(O)N(alkyl)₂, -OC(O)(alkyl), -OC(O)O(alkyl), -OC(O)NH₂, -OC(O)NH(alkyl), -OC(O)N(alkyl)₂, -NHC(O)H, -NHC(O)(alkyl), -NHC(O)O(alkyl), -NHC(O)NH₂, -NHC(O)NH(alkyl), -NHC(O)N(alkyl)₂, -SO₂NH₂, -SO₂NH(alkyl), -SO₂N(alkyl)₂, and R⁴⁰, in which R³⁰ is alkyl or alkyl substituted with one substituent selected from the group consisting of halo, -O(alkyl), and -S(alkyl), and R⁴⁰ is furyl, imidazolyl, indazolidinyl, isoquinolyl, isothiazolyl, isoxazolyl, morpholinyl, naphthyl, naphthyridyl, 1,2,3-oxadiazolyl, oxazolyl, phenyl, piperidinyl, piperazinyl, pyrazinyl, pyrazolyl, pyridyl, pyrimidinyl, pyrrolidinyl, pyrrolyl, quinazolyl, quinolyl, quinoxalyl, tetrazolyl, 1,2,3-thiadiazolyl, 1,3,4-thiadiazolyl, thiazolyl, thienyl, 1,2,3-triazolyl, or thiomorpholinyl, in which each R⁴⁰ moiety is unsubstituted or substituted with one, two, or three substituents independently selected from the group consisting of alkyl, alkenyl, alkynyl, cycloalkyl, halo, =O, -CN, -OH, -SH, -NO₂, -CF₃, -CH₂CF₃, -CF₂CF₃, -OCF₃, -OCH₂CF₃, -OCF₂CF₃, -O(alkyl), -S(alkyl), -S(O)(alkyl), -SO₂(alkyl), -C(O)H, -C(O)(alkyl), -C(O)OH, -C(O)O(alkyl), -NH₂, -NH(alkyl), -N(alkyl)₂, -C(O)NH₂, -C(O)NH(alkyl), -C(O)N(alkyl)₂, -OC(O)(alkyl), -OC(O)O(alkyl), -OC(O)NH₂, -OC(O)NH(alkyl), -OC(O)N(alkyl)₂, -NHC(O)H, -NHC(O)(alkyl), -NHC(O)O(alkyl), -NHC(O)NH₂, -NHC(O)NH(alkyl), -NHC(O)N(alkyl)₂, -SO₂NH₂, -SO₂NH(alkyl), and -SO₂N(alkyl)₂.

Substituted cycloalkyl, cycloalkenyl, and heterocyclyl moieties are those moieties substituted with one, two, or three substituents independently selected from the group consisting of alkyl, halo, -CN, -OH, -NH₂, -CF₃, -OR³⁰, -SR³⁰, -S(O)(alkyl), -SO₂(alkyl), -C(O)H, -C(O)(alkyl), -C(O)OH, -C(O)O(alkyl), -NH(alkyl), -N(alkyl)₂, -C(O)NH₂, -C(O)NH(alkyl), -C(O)N(alkyl)₂, and R⁴⁰, in which the phenyl is unsubstituted or substituted with one, two, or three substituents independently selected from the group consisting of halo, -CN, -OH, -NH₂, and -CF₃.

"Hydroxyl protecting moiety" means selectively introducible and removable moieties which protect -OH moieties against undesirable side reactions. Hydroxyl protecting moieties include 4-nitrobenzyloxycarbonyl, 4-bromobenzyloxycarbonyl, 4-methoxybenzyloxycarbonyl, 3,4-dimethoxybenzyloxycarbonyl, tert-butoxycarbonyl, diphenylmethoxycarbonyl, 2,2,2-trichloroethoxycarbonyl, 2,2,2-tribromoethoxycarbonyl, 2-(trimethylsilyl)ethoxycarbonyl, 2-(phenylsulfonyl)ethoxycarbonyl, allyloxycarbonyl, acetyl, chloroacetyl, dichloroacetyl, trichloroacetyl, trifluoroacetyl, methoxyacetyl, phenoxyacetyl, pivaloyl, propionyl, 2-methylpropionyl, benzoyl, tert-butyl, 2,2,2-trichloroethyl, 2-trimethylsilylethyl, 1,1-dimethyl-2-propenyl, 3-methyl-3-butenyl, para-methoxybenzyl, 3,4-dimethoxybenzyl, diphenylmethyl, triphenylmethyl, tetrahydrofuryl, benzyloxymethyl, 2-methoxyethoxymethyl, 2,2,2-trichloroethoxymethyl, 2-(trimethylsilyl)ethoxymethyl, methanesulfonyl, para-toluenesulfonyl, trimethylsilyl, triethylsilyl, triisopropylsilyl, diethylisopropylsilyl, tert-butyldimethylsilyl, tert-butyldiphenylsilyl, diphenylmethylsilyl, and tert-butylmethoxyphenylsilyl.

These variable moieties may combine to provide a seventh embodiment of this invention, which embodiment is directed to compounds having formula (I), formula (I)-f, formula (I)-g, formula (II), formula (II)-f, and formula (II)-g, and pharmaceutically acceptable salts, prodrugs, and salts of prodrugs thereof, in which
R¹ is -OH, -OR⁹, -OC(O)OR⁹, -OC(O)NH₂, -OC(O)NHR¹⁰, or -OC(O)NR¹⁰R¹¹;
R² is hydrogen or R^{p}, in which R^{p} is a hydroxyl protecting moiety;
one of R³ and R⁴ is hydrogen, and the other is -OH, -OR¹³, -OC(O)OR¹³, -NH₂, -NHC(O)OR¹⁴, -NHR¹⁵, -NR¹⁵R¹⁶, -OC(O)NH₂, -OC(O)NHR¹⁵, -OC(O)NR¹⁵R¹⁶, -N(R¹⁷)C(O)NH₂, -N(R¹⁷)C(O)NHR¹⁵, or -N(R¹⁷)C(O)NR¹⁵R¹⁶; or
R³ and R⁴ together are =O or =NOR¹⁹;
one of R⁵ and R⁶ is hydrogen, and the other is -OH, -OR²⁰, -OC(O)OR²⁰, -NH₂, -NHC(O)OR¹⁴, -NHR²¹, -NR²¹R²², -OC(O)NH₂, -OC(O)NHR²¹, -OC(O)NR²¹R²², -N(R²³)C(O)NH₂, -N(R²³)C(O)NHR²¹, or -N(R²³)C(O)NR²¹R²²; or
R⁵ and R⁶ together are =O;
R⁷ is hydrogen and R⁸ is -OH, -OR²⁵, -OC(O)R²⁵, -OC(O)OR²⁵, -OC(O)NH₂, -OC(O)NHR²⁶, or -OC(O)NR²⁶R²⁷; or
R⁷ and R⁸ together are =O;
R⁹, R¹³, R¹⁹, R²⁰, and R²⁵ are independently alkyl, cycloalkyl, -(CH₂)alkenyl, -(CH₂)alkynyl, alkyl substituted with one substituent selected from the group consisting of cycloalkyl, aryl, heteroaryl, and heterocyclyl, -(CH₂)alkenyl substituted with one substituent selected from the group consisting of cycloalkyl, aryl, heteroaryl, and heterocyclyl, or -(CH₂)alkynyl substituted with one substituent selected from the group consisting of cycloalkyl, aryl, heteroaryl, and heterocyclyl;
R¹⁰, R¹¹, R¹⁵, R¹⁶, R²¹, R²², R²⁶, and R²⁷ are independently alkyl, cycloalkyl, -(CH₂)alkenyl, -(CH₂)alkynyl, aryl, heteroaryl, heterocyclyl, alkyl substituted with one substituent selected from the group consisting of cycloalkyl, aryl, heteroaryl, heterocyclyl, -NH₂, -NHR³¹, and -NR³¹R³², -(CH₂)alkenyl substituted with one substituent selected from the group consisting of cycloalkyl, aryl, heteroaryl, heterocyclyl, -NH₂, -NHR³¹, and -NR³¹R³², or -(CH₂)alkynyl substituted with one substituent selected from the group consisting of cycloalkyl, aryl, heteroaryl, heterocyclyl, -NH₂, -NHR³¹, and -NR³¹R³²;
R¹⁴ is alkyl or alkyl substituted with one or two independently selected aryl substituents;
R¹⁷ and R²³ are independently hydrogen or alkyl;
R³¹ and R³² are independently alkyl, cycloalkyl, aryl, heteroaryl, heterocyclyl, -(CH₂)alkenyl, -(CH₂)alkynyl, alkyl substituted with one substituent selected from the group consisting of cycloalkyl, aryl, heteroaryl, heterocyclyl, -NH₂, -NH(alkyl), and -N(alkyl)₂, -(CH₂)alkenyl substituted with one substituent selected from the group consisting of cycloalkyl, aryl, heteroaryl, heterocyclyl, -NH₂, -NH(alkyl), and -N(alkyl)₂, or -(CH₂)alkynyl substituted with one substituent selected from the group consisting of cycloalkyl, aryl, heteroaryl, heterocyclyl, -NH₂, -NH(alkyl), and -N(alkyl)₂; and
X¹ is hydrogen, fluoride, chloride, or bromide;
compounds having formula (I), formula (I)-f, formula (I)-g, formula (II), formula (II)-f, or formula (II)-g, and pharmaceutically acceptable salts, prodrugs, and salts of prodrugs thereof, in which
R¹ is -OH or -OR⁹;
R² is hydrogen or R^{P}, in which R^{P} is a hydroxyl protecting moiety;
one of R³ and R⁴ is hydrogen, and the other is -OH, -OR¹³, -OC(O)OR¹³, -NH₂, -NHC(O)OR¹⁴, -NHR¹⁵, -NR¹⁵R¹⁶, -OC(O)NH₂, -OC(O)NHR¹⁵, -OC(O)NR¹⁵R¹⁶, -N(R¹⁷)C(O)NH₂, -N(R¹⁷)C(O)NHR¹⁵, or -N(R¹⁷)C(O)NR¹⁵R¹⁶; or
R³ and R⁴ together are =O;
one of R⁵ and R⁶ is hydrogen, and the other is -OH, -OR²⁰, -OC(O)OR²⁰, -NH₂, -NHC(O)OR¹⁴, -NHR²¹, -NR²¹R²², -OC(O)NH₂, -OC(O)NHR²¹, -OC(O)NR²¹R²², -N(R²³)C(O)NH₂, -N(R²³)C(O)NHR²¹, or -N(R²³)C(O)NR²¹R²²; or
R⁵ and R⁶ together are =O;
R⁷ is hydrogen and R⁸ is -OH, -OR²⁵, -OC(O)R²⁵, -OC(O)OR²⁵, -OC(O)NH₂, -OC(O)NHR²⁶, or -OC(O)NR²⁶R²⁷; or
R⁷ and R⁸ together are =0;
R⁹, R¹³, R²⁰, and R²⁵ are independently alkyl, cycloalkyl, -(CH₂)alkenyl, -(CH₂)alkynyl, alkyl substituted with one substituent selected from the group consisting of cycloalkyl, aryl, heteroaryl, and heterocyclyl, -(CH₂)alkenyl substituted with one substituent selected from the group consisting of cycloalkyl, aryl, heteroaryl, and heterocyclyl, or -(CH₂)alkynyl substituted with one substituent selected from the group consisting of cycloalkyl, aryl, heteroaryl, and heterocyclyl;
R¹⁵, R¹⁶, R²¹, R²², R²⁶, and R²⁷ are independently alkyl, cycloalkyl, -(CH₂)alkenyl, -(CH₂)alkynyl, aryl, heteroaryl, heterocyclyl, alkyl substituted with one substituent selected from the group consisting of cycloalkyl, aryl, heteroaryl, heterocyclyl, -NH₂, -NHR³¹, and -NR³¹R³², -(CH₂)alkenyl substituted with one substituent selected from the group consisting of cycloalkyl, aryl, heteroaryl, heterocyclyl, -NH₂, -NHR³¹, and -NR³¹R³², or -(CH₂)alkynyl substituted with one substituent selected from the group consisting of cycloalkyl, aryl, heteroaryl, heterocyclyl, -NH₂, -NHR³¹, and -NR³¹R³²;
R¹⁴ is alkyl or alkyl substituted with one or two independently selected aryl substituents;
R¹⁷ and R²³ are independently hydrogen or alkyl;
R³¹ and R³² are independently alkyl, cycloalkyl, aryl, heteroaryl, heterocyclyl, -(CH₂)alkenyl, -(CH₂)alkynyl, alkyl substituted with one substituent selected from the group consisting of cycloalkyl, aryl, heteroaryl, heterocyclyl, -NH₂, -NH(alkyl), and -N(alkyl)₂, -(CH₂)alkenyl substituted with one substituent selected from the group consisting of cycloalkyl, aryl, heteroaryl, heterocyclyl, -NH₂, -NH(alkyl), and -N(alkyl)₂, or -(CH₂)alkynyl substituted with one substituent selected from the group consisting of cycloalkyl, aryl, heteroaryl, heterocyclyl, -NH₂, -NH(alkyl), and -N(alkyl)₂; and
X¹ is hydrogen, fluoride, chloride, or bromide;
compounds having formula (I), formula (I)-f, formula (I)-g, formula (II), formula (II)-f, and formula (II)-g, and pharmaceutically acceptable salts, prodrugs, and salts of prodrugs thereof, in which
R¹ is -OH or -OR⁹;
R² is hydrogen;
one of R³ and R⁴ is hydrogen, and the other is -OH, -OR¹³, -NH₂, -NHC(O)OR¹⁴, -NHR¹⁵, -NR¹⁵R¹⁶, -OC(O)NH₂, -OC(O)NHR¹⁵, or -OC(O)NR¹⁵R¹⁶; or
R³ and R⁴ together are =O;
one of R⁵ and R⁶ is hydrogen, and the other is -OH, -OR²⁰, -OC(O)OR²⁰, -OC(O)NH₂, -OC(O)NHR²¹, or -OC(O)NR²¹R²²; or
R⁵ and R⁶ together are =O;
R⁷ is hydrogen and R⁸ is -OH, -OR²⁵, -OC(O)R²⁵, -OC(O)OR²⁵, -OC(O)NH₂, -OC(O)NHR²⁶, or -OC(O)NR²⁶R²⁷; or
R⁷ and R⁸ together are =0;
R⁹, R¹³, R²⁰, and R²⁵ are independently alkyl, -(CH₂)alkenyl, -(CH₂)alkynyl, alkyl substituted with one substituent selected from the group consisting of aryl, heteroaryl, and heterocyclyl, -(CH₂)alkenyl substituted with one substituent selected from the group consisting of aryl, heteroaryl, and heterocyclyl, or -(CH₂)alkynyl substituted with one substituent selected from the group consisting of aryl, heteroaryl, and heterocyclyl;
R¹⁵, R¹⁶, R²¹, R²², R²⁶, and R²⁷ are independently alkyl, cycloalkyl, -(CH₂)alkenyl, -(CH₂)alkynyl, aryl, heteroaryl, heterocyclyl, alkyl substituted with one substituent selected from the group consisting of aryl, heteroaryl, heterocyclyl, -NH₂, -NHR³¹, and -NR³¹R³², -(CH₂)alkenyl substituted with one substituent selected from the group consisting of aryl, heteroaryl, heterocyclyl, -NH₂, -NHR³¹, and -NR³¹R³², or -(CH₂)alkynyl substituted with one substituent selected from the group consisting of aryl, heteroaryl, heterocyclyl, -NH₂, -NHR³¹, and -NR³¹R³²;
R¹⁴ is alkyl or alkyl substituted with phenyl;
R³¹ and R³² are independently alkyl, -(CH₂)alkenyl, -(CH₂)alkynyl, alkyl substituted with one substituent selected from the group consisting of aryl and heteroaryl, -(CH₂)alkenyl substituted with one substituent selected from the group consisting of aryl and heteroaryl, or -(CH₂)alkynyl substituted with one substituent selected from the group consisting of aryl and heteroaryl; and
X¹ is hydrogen, fluoride, chloride, or bromide;
compounds having formula (I), formula (I)-f, formula (I)-g, formula (II), formula (II)-f, and formula (II)-g, and pharmaceutically acceptable salts, prodrugs, and salts of prodrugs thereof, in which
R¹ is -OH or -OR⁹;
R² is hydrogen;
one of R³ and R⁴ is hydrogen, and the other is -OH, -NH₂, -NHR¹⁵, -NR¹⁵R¹⁶ or -NHC(O)OR¹⁴; or
R³ and R⁴ together are =O;
R⁵ is hydrogen, and R⁶ is -OH, -OC(O)NH₂, -OC(O)NHR²¹, or -OC(O)NR²¹R²²;
R⁷ is hydrogen and R⁸ is -OH, -OR²⁵, -OC(O)R²⁵, -OC(O)NH₂, -OC(O)NHR²⁶, or -OC(O)NR²⁶R²⁷;
R²¹ and R²² are independently methyl, ethyl, propyl, butyl, prop-2-enyl, or prop-2-ynyl, each of which is independently unsubstituted or substituted with one substituent selected from the group consisting of -NH₂ and -NHR³¹;
R¹⁵, R¹⁶, R²⁶ and R²⁷ are independently methyl, ethyl, propyl, butyl, prop-2-enyl, prop-2-ynyl, cyclopropyl, cyclopentyl, cyclohexyl, cyclopropyl substituted with phenyl, phenyl substituted with two independently selected halo substituents, or methyl, ethyl, propyl, butyl, prop-2-enyl, or prop-2-ynyl, each of which is substituted with one substitutent selected from the group consisting of (4,5-dihydroisoxazol-5-yl), phenyl, pyridyl, pyrimidinyl, thienyl, isoxazolyl, oxazolyl, quinolyl and isoquinolyl, in which substituent is unsubstituted or substituted with one substituent selected from the group consisting of -F, -Cl, -Br, methyl, -OH, (methyl)O-, phenyl, pyridyl, pyrimidinyl, thienyl and isoxazolyl;
R⁹ and R²⁵ are independently methyl, ethyl, propyl, butyl, prop-2-enyl, or prop-2-ynyl, each of which is independently unsubstituted or substituted with one substituent selected from the group consisting of thienyl, isoxazolyl, 4,5-dihydroisoxazol-5-yl, phenyl, pyridyl, pyrimidinyl, quinolyl, and isoquinolyl, in which each substituent is independently unsubstituted or substituted with one substituent selected from the group consisting of phenyl, pyridyl, pyrimidinyl, thienyl, isoxazolyl, quinolyl, isoquinolyl, and phenyl substituted with one substituent selected from the group consisting of methyl, -OH, (methyl)O-, -F, -Cl, and -Br;
R¹⁴ is tert-butyl or phenylmethyl;
R³¹ is methyl, ethyl, or propyl, each of which is independently unsubstituted or substituted with one substituent selected from the group consisting of phenyl, pyridyl, quinolyl, isoquinolyl, thienyl, pyrimidinyl, isoxazolyl, and oxazolyl, in which each substituent is unsubstituted or substituted with one or two or three substituents independently selected from the group consisting of -F, -Cl, -Br, -I, methyl, -OH, and (methyl)O-; and
X¹ is hydrogen, fluoride, chloride, or bromide; and
compounds having formula (I), formula (I)-f, formula (I)-g, formula (II), formula (II)-f, or formula (II)-g, and pharmaceutically acceptable salts, prodrugs, and salts of prodrugs thereof, in which
R¹ is -OH, (methyl)O-, (ethyl)O-, (prop-2-ynyl)O-, (prop-2-enyl)O-, (phenylmethyl)O-, (3-(5-pyridin-2-ylthien-2-yl)prop-2-ynyl)O-, (3-(quinolin-3-yl)prop-2-enyl)O-, (3-(3-(pyridin-2-yl)isoxazol-5-yl)prop-2-ynyl)O-, or (3-(5-(pyrimidin-2-yl)thien-2-yl)prop-2-ynyl)O-;
R² is hydrogen;
R³ is hydrogen, and R⁴ is -OH, -NH₂, (tert-butyl)OC(O)NH-, (phenylmethyl)OC(O)NH-, (methyl)NH-, (methyl)₂N-, (ethyl)NH-, (propyl)NH-, (butyl)NH-, (prop-2-ynyl)NH-, (prop-2-enyl)NH-, (methyl)(phenylmethyl)N-, (3-(quinolin-3-yl)prop-2-enyl)NH-, (3-(3-pyridin-2-ylisoxazol-5-yl)prop-2-ynyl)NH-(3-(5-(pyrimidin-2-yl)thien-2-yl)prop-2-ynyl)NH-(3-(quinolin-3-yl)propyl)NH- (3-(quinolin-3-yl)butyl)NH- or (4-(quinolin-3-yl)butyl)NH-; or
R³ and R⁴ together are =O;
R⁵ is hydrogen, and R⁶ is (2-aminoethyl)NHC(O)O-, (2-(dimethylamino)ethyl)NHC(O)O-, (3-aminopropyl)NHC(O)O-, (4-aminobutyl)NHC(O)O-, (2-((1-(2-methoxyphenyl)ethyl)amino)ethyl)NHC(O)O-, (2-((quinolin-3-ylmethyl)amino)ethyl)NHC(O)O-, (2-((quinolin-4-ylmethyl)amino)ethyl)NHC(O)O-, or (2-((pyridin-2-ylmethyl)amino)ethyl)NHC(O)O-;
R⁷ is hydrogen;
R⁸ is -OH, (methyl)O-, (ethyl)O-, (propyl)O-, (prop-2-ynyl)O-, (prop-2-enyl)O-, (3-(5-(pyridin-2-yl)thien-2-yl)prop-2-ynyl)O-, (3-(quinolin-3-yl)prop-2-enyl)O-, (3-(3-(pyridin-2-yl)isoxazol-5-yl)prop-2-ynyl)O-, (3-(5-(pyrimidin-2-yl)thien-2-yl)prop-2-ynyl)O-, (3-phenyl-4,5-dihydroisoxazol-5-yl)CH₂O-, (3-(pyridin-2-yl)-4,5-dihydroisoxazol-5-yl)CH₂O-, (3-(4-fluorophenyl)-4,5-dihydroisoxazol-5-yl)CH₂O-, ((pyridin-2-yl)methyl)C(O)O-, (2-(pyrictin-3-yl)ethyl)C(O)O-, (ethyl)NHC(O)O-, (propyl)NHC(O)O-, (isopropyl)NHC(O)O-, (3,5-dichlorophenyl)NHC(O)O-, (cyclopropyl)NHC(O)O-, (cyclopentyl)NHC(O)O-, (cyclohexyl)NHC(O)O-, (2-phenylcyclopropyl)NHC(O)O-, (phenylmethyl)NHC(O)O-, (2-fluorophenylmethyl)NHC(O)O-, (3-fluorophenylmethyl)NHC(O)O-, (4-fluorophenylmethyl)NHC(O)O-, ((4-methylphenyl)methyl)NHC(O)O-, ((4-methoxyphenyl)methyl)NHC(O)O-, ((pyridin-2-yl)methyl)NHC(O)O-, ((pyridin-3-yl)methyl)NHC(O)O-, ((pyridin-4-yl)methyl)NHC(O)O-, ((3-(phenyl)-4,5-dihydroisoxazol-5-yl)methyl)NHC(O)O-, (2-(pyridin-2-yl)ethyl)NHC(O)O-, (2-(pyridin-3-yl)ethyl)NHC(O)O-, (2-(pyridin-4-yl)ethyl)NHC(O)O-, or (quinolin-4-ylmethyl)NHC(O)O-; and
X¹ is hydrogen or fluoride, chloride, or bromide.

An example of an R¹ moiety for the practice of this invention using compounds having formula (I), formula (I)-f, formula (I)-g, formula (II), formula (II)-f, or formula (II)-g, is -OH.

An example of R² moiety for the practice of this invention using compounds having formula (I), formula (I)-f, formula (I)-g, formula (II), formula (II)-f, or formula (II)-g, is hydrogen.

Examples of R³ and R⁴ moieties for the practice of this invention using compounds having formula (I), formula (I)-f, or formula (I)-g are hydrogen and -NH₂, respectively, or taken together are =O.

An example of an R³ moiety for the practice of this invention using compounds having formula (II), formula (II)-f, or formula (II)-g is hydrogen.

Examples of R⁴ moieties for the practice of this invention using compounds having formula (II), formula (II)-f, or formula (II)-g are -NH₂, (tert-butyl)OC(O)NH-, and (phenylmethyl)OC(O)NH-.

An example of an R⁵ moiety for the practice of this invention using compounds having formula (I), formula (I)-f, or formula (I)-g is hydrogen.

Examples of R⁶ moieties for the practice of this invention using compounds having formula (I), formula (I)-f, or formula (I)-g are (2-((1-(2-methoxyphenyl)ethyl)amino)ethyl)NHC(O)O-, (2-aminoethyl)NHC(O)O-, (2-((quinolin-3-ylmethyl)amino)ethyl)NHC(O)O-, (2-((quinolin-4-ylmethyl)amino)ethyl)NHC(O)O-, and (2-((pyridin-2-ylmethyl)amino)ethyl)NHC(O)O-.

An example of an R⁷ moiety for the practice of this invention using compounds having formula (I), formula (I)-f, formula (I)-g, formula (II), formula (II)-f, or formula (II)-g is hydrogen.

Examples of an R⁸ moiety for the practice of this invention using compounds having formula (II), formula (II)-f, or formula (II)-g are -OH, (propyl)O-, (3-phenyl-4,5-dihydroisoxazol-5-yl)methoxy, (3-(pyridin-2-yl)-4,5-dihydroisoxazol-5-yl)methoxy, (3-(4-fluorophenyl)-4,5-dihydroisoxazol-5-yl)methoxy, ((pyridin-2-yl)methyl)C(O)O-, (2-(pyridin-3-yl)ethyl)C(O)O-, (propyl)NHC(O)O-, (isopropyl)NHC(O)O-, (cyclopentyl)NHC(O)O-, (cyclohexyl)NHC(O)O-, (2-phenylcyclopropyl)NHC(O)O-, (3,5-dichlorophenyl)NHC(O)O-, (phenylmethyl)NHC(O)O-, ((2-fluorophenyl)methyl)NHC(O)O-, ((3-fluorophenyl)methyl)NHC(O)O-, ((4-fluorophenyl)methyl)NHC(O)O-, ((4-methylphenyl)methyl)NHC(O)O-, ((4-methoxyphenyl)methyl)NHC(O)O-, ((pyridin-2-yl)methyl)NHC(O)O-, ((pyridin-3-yl)methyl)NHC(O)O-, ((pyridin-4-yl)methyl)NHC(O)O-, ((3-(phenyl)-4,5-dihydroisoxazol-5-yl)methyl)NHC(O)O-, (2-(pyridin-2-yl)ethyl)NHC(O)O-, (2-(pyridin-3-yl)ethyl)NHC(O)O-, and (2-(pyridin-4-yl)ethyl)NHC(O)O-.

An example of an X¹ moiety for the practice of this invention using compounds having formula (I), formula (I)-f, formula (I)-g, formula (II), formula (II)-f, or formula (I)-g, is hydrogen.

These specific moieties of the compounds may combine with the fixed moieties thereof to form an eighth embodiment of this invention, which embodiment is directed to compounds, and salts, prodrugs, and salts of prodrugs thereof, which are useful as antibacterials, the compounds having formula (I) 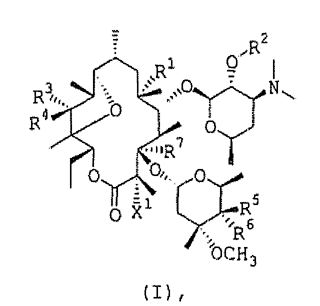 formula (I)-f, 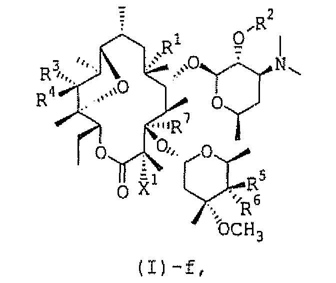 and formula (I)-g, 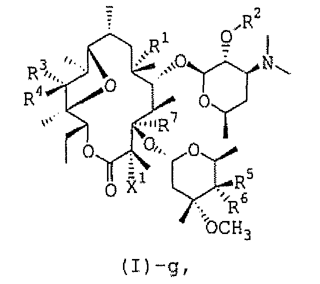 in which
R¹ is -OH; R² is hydrogen; R³ is hydrogen and R⁴ is -NH₂, or R³ and R⁴ together are =O; R⁵ and R⁷ are hydrogen; R⁶ is -OC(O)NHR²¹; R²¹ is alkyl substituted with one substituent selected from the group consisting of -NH₂ and -NHR³¹; R³¹ is alkyl substituted with one substituent selected from the group consisting of phenyl and pyridyl, in which the phenyl is substituted with -O(alkyl) and the pyridyl is unfused or fused with phenyl; and X¹ is hydrogen;

compounds, and salts, prodrugs, and salts of prodrugs thereof, which are useful as antibacterials, the compounds having formula (I) 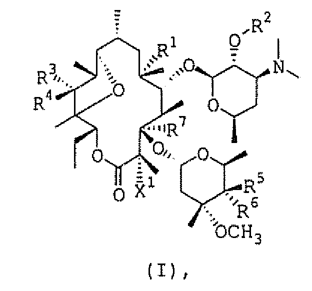 formula (I)-f, 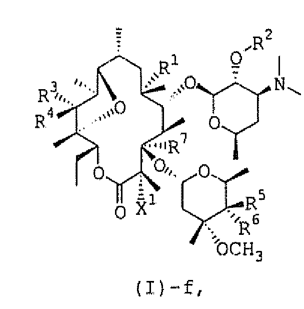 and formula (I)-g, 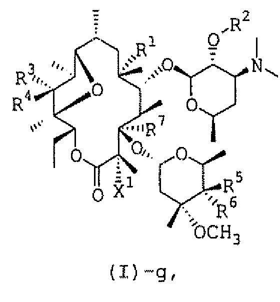 in which
R¹ is -OH; R² is hydrogen; R³ is hydrogen and R⁴ is -NH₂, or R³ and R⁴ together are =O; R⁵ and R⁷ are hydrogen; R⁶ is -OC(O)NHR²¹; R²¹ is C₂-alkyl substituted with one substituent selected from the group consisting of -NH₂ and -NHR³¹; R³¹ is C₁-C₂-alkyl substituted with one substituent selected from the group consisting of phenyl and pyridyl, in which the phenyl is substituted with (methyl)O- and the pyridyl is unfused or fused with phenyl; and X¹ is hydrogen;

compounds, and salts, prodrugs, and salts of prodrugs thereof, which are useful as antibacterials, the compounds having formula (I), formula (I)-f, or formula (I)-g, in which R¹ is -OH; R² is hydrogen; R³ is hydrogen; R⁴ is -NH₂; R⁵ and R⁷ are hydrogen; R⁶ is (2-((1-(2-methoxyphenyl)ethyl)amino)ethyl)NHC(O)O-, (2-aminoethyl)NHC(O)O-, (2-((quinolin-3-ylmethyl)amino)ethyl)NHC(O)O-, (2-((quinolin-4-ylmethyl)amino)ethyl)NHC(O)O-, or (2-((pyridin-2-ylmethyl)amino)ethyl)NHC(O)O-; and
X¹ is hydrogen;
compounds having formula (I), formula (I)-f, or formula (I)-g, and salts, prodrugs, and salts of prodrugs thereof, which are useful as antibacterials, in which R¹ is -OH; R² is hydrogen; R³ and R⁴ together are =0; R⁵ and R⁷ are hydrogen; R⁶ is (2-((1-(2-methoxyphenyl)ethyl)amino)ethyl)NHC(O)O-, (2-aminoethyl)NHC(O)O-, (2-((quinolin-3-ylmethyl)amino)ethyl)NHC(O)O-, (2-((quinolin-4-ylmethyl)amino)ethyl)NHC(O)O-, or (2-((pyridin-2-ylmethyl)amino)ethyl)NHC(O)O-; and X¹ is hydrogen;
compounds, and salts, prodrugs, and salts of prodrugs thereof, which are useful as antibacterials, the compounds having formula (II) 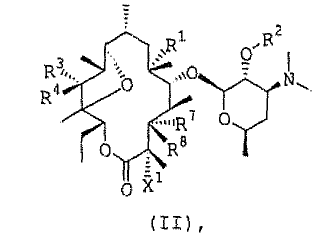 formula (II)-f, 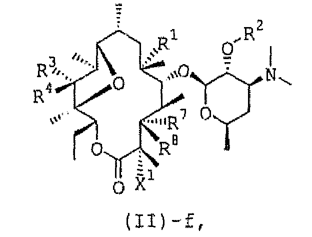 or formula (II)-g, 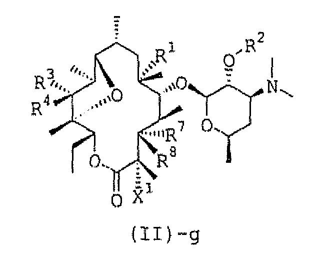 in which
R¹ is -OH; R² is hydrogen; R³ and R⁷ are hydrogen; R⁴ is -NH₂ or -NHC(O)OR¹⁴; R⁸ is -OH, -OR²⁵, -OC(O)R²⁵, or -OC(O)NHR²⁶; R¹⁴ is alkyl substituted with phenyl; R²⁵ is alkyl or alkyl substituted with one substituent selected from the group consisting of pyridyl and 4,5-dihydroisoxazolyl, in which the 4,5-dihydroisoxazolyl is substituted with one substituent selected from the group consisting of pyridyl and phenyl, in which the phenyl is unsubstituted or substituted with one halo substituent; R²⁶ is alkyl, cycloalkyl, cycloalkyl substituted with phenyl, phenyl substituted with two independently selected halo substituents, or alkyl substituted with one substituent selected from the group consisting of phenyl, pyridyl, and 4,5-dihydroisoxazolyl, in which the phenyl is unsubstituted or substituted with one substituent selected from the group consisting of alkyl, halo and -O(alkyl), and the 4,5-dihydroisoxazolyl is substituted with phenyl; and X¹ is hydrogen;
compounds, and salts, prodrugs, and salts of prodrugs thereof, which are useful as antibacterials, the compounds having formula (II) 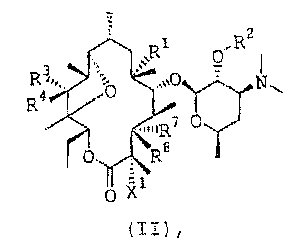 formula (II)-f, 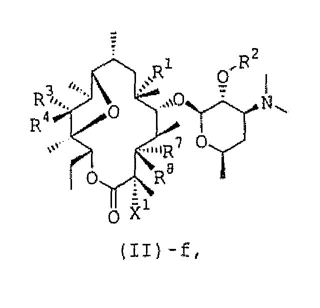 or formula (II)-g, 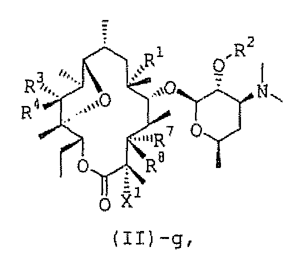 in which
R¹ is -OH; R², R³ and R⁷ are hydrogen; R⁴ is -NH₂ or -NHC(O)OR¹⁴; R⁸ is -OH, -OR²⁵, -OC(O)R²⁵, or -OC(O)NHR²⁶; R¹⁴ is phenylmethyl; R²⁵ is C₃-alkyl or C₁-C₂-alkyl substituted with one substituent selected from the group consisting of pyridyl and 4,5-dihydroisoxazolyl, in which the 4,5-dihydroisoxazolyl is substituted with one substituent selected from the group consisting of pyridyl and phenyl, in which the phenyl is unsubstituted or substituted with one halo substituent; R²⁶ is C₃-alkyl, C₅-C₆-cycloalkyl, C₃-cycloalkyl substituted with phenyl, phenyl substituted with two independently selected halo substituents, or C₁-C₂-alkyl substituted with one substituent selected from the group consisting of phenyl, pyridyl, and 4,5-dihydroisoxazolyl, in which the phenyl is unsubstituted or substituted with one substituent selected from the group consisting of methyl, halo and (methyl)O-, and the 4,5-dihydroisoxazolyl is substituted with phenyl; and X¹ is hydrogen;
compounds having formula (II), formula (II)-f, or formula (II)-g, and salts, prodrugs, and salts of prodrugs thereof, which are useful as antibacterials, in which R¹ is -OH; R², R³ and R⁷ are hydrogen; R⁴ is -NH₂; R⁸ is -OH, (propyl)O-, (3-phenyl-4,5-dihydroisoxazol-5-yl)methoxy, (3-(pyridin-2-yl)-4,5-dihydroisoxazol-5-yl)methoxy, (3-(4-fluorophenyl)-4,5-dihydroisoxazol-5-yl)methoxy, ((pyridin-2-yl)methyl)C(O)O-, (2-(pyridin-3-yl)ethyl)C(O)O-, (propyl)NHC(O)O-, (isopropyl)NHC(O)O-, (cyclopentyl)NHC(O)O-, (cyclohexyl)NHC(O)O-, (2-phenylcyclopropyl)NHC(O)O-, (3,5-dichlorophenyl)NHC(O)O-, (phenylmethyl)NHC(O)O-, ((2-fluorophenyl)methyl)NHC(O)O-, ((3-fluorophenyl)methyl)NHC(O)O-, ((4-fluorophenyl)methyl)NHC(O)O-, ((4-methylphenyl)methyl)NHC(O)O-, ((4-methoxyphenyl)methyl)NHC(O)O-, ((pyridin-2-yl)methyl)NHC(O)O-, ((pyridin-3-yl)methyl)NHC(O)O-, ((pyridin-4-yl)methyl)NHC(O)O-, ((3-(phenyl)-4,5-dihydroisoxazol-5-yl)methyl)NHC(O)O-, (2-(pyridin-2-yl)ethyl)NHC(O)O-, (2-(pyridin-3-yl)ethyl)NHC(O)O-, or (2-(pyridin-4-yl)ethyl)NHC(O)O-; and X¹ is hydrogen;
compounds having formula (II), formula (II)-f, or formula (II)-g, and salts, prodrugs, and salts of prodrugs thereof, which are useful as antibacterials, in which R¹ is -OH; R², R³ and R⁷ are hydrogen; R⁴ is (phenylmethyl)OC(O)NH-; R⁸ is -OH, (propyl)O-, (3-phenyl-4,5-dihydroisoxazol-5-yl)CH₂O-, (3-(pyridin-2-yl)-4,5-dihydroisoxazol-5-yl)CH₂O-, (3-(4-fluorophenyl)-4,5-dihydroisoxazol-5-yl)CH₂O-, ((pyridin-2-yl)methyl)C(O)O-, (2-(pyridin-3-yl)ethyl)C(O)O-, (propyl)NHC(O)O-, (isopropyl)NHC(O)O-, (cyclopentyl)NHC(O)O-, (cyclohexyl)NHC(O)O-, (2-phenylcyclopropyl)NHC(O)O-, (3,5-dichlorophenyl)NHC(O)O-, (phenylmethyl)NHC(O)O-, ((2-fluorophenyl)methyl)NHC(O)O-, ((3-fluorophenyl)methyl)NHC(O)O-, ((4-fluorophenyl)methyl)NHC(O)O-, ((4-methylphenyl)methyl)NHC(O)O-, ((4-methoxyphenyl)methyl)NHC(O)O-, ((pyridin-2-yl)methyl)NHC(O)O-, ((pyridin-3-yl)methyl)NHC(O)O-, ((pyridin-4-yl)methyl)NHC(O)O-, ((3-(phenyl)-4,5-dihydroisoxazol-5-yl)methyl)NHC(O)O-, (2-(pyridin-2-yl)ethyl)NHC(O)O-, (2-(pyridin-3-yl)ethyl)NHC(O)O-, or (2-(pyridin-4-yl)ethyl)NHC(O)O-; and X¹ is hydrogen; and
compounds, and salts, prodrugs, and salts of prodrugs thereof, which are useful as antibacterials, including
(1-(S or R),2R,5R,6S,7S,8R,9S,11R,12S,13S)-2-ethyl-9-hydroxy-1,5,7,9,11,13-hexamethyl-4,14-dioxo-8-((3,4,6-trideoxy-3-(dimethylamino)-β-D-xylo-hexopyranosyl)oxy)-3,15-dioxabicyclo(10.2.1)pentadec-6-yl 4-O-(((2-aminoethyl)amino)carbonyl)-2,6-dideoxy-3-C-methyl-3-O-methyl-α-L-ribo-hexopyranoside;
(1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy-3,15-dioxabicyclo(10.2.1)pentadec-6-yl 2,6-dideoxy-4-O-(((2-((1-(2-methoxyphenyl)ethyl)amino)ethyl)amino)carbonyl)-3-C-methyl-3-O-methyl-α-L-ribo-hexopyranoside;
(1-(S or R),2R,5R,6S,7S,8R,9S,11R,12S,13S)-2-ethyl-9-hydroxy-1,5,7,9,11,13-hexamethyl-4,14-dioxo-8-((3,4,6-trideoxy-3-(dimethylamino)-β-D-xylo-hexopyranosyl)oxy)-3,15-dioxabicyclo(10.2.1)pentadec-6-yl 2,6-dideoxy-3-C-methyl-3-O-methyl-4-O-(((2-((quinolin-3-ylmethyl)amino)ethyl)amino)carbonyl)-α-L-ribohexopyranoside;
(1-(S or R),2R,5R,6S,7S,8R,9S,11R,12S,13S)-2-ethyl-9-hydroxy-1,5,7,9,11,13-hexamethyl-4,14-dioxo-8-((3,4,6-trideoxy-3-(dimethylamino)-β-D-xylo-hexopyranosyl)oxy)-3,15-dioxabicyclo(10.2.1)pentadec-6-yl 2,6-dideoxy-3-C-methyl-3-O-methyl-4-O-(((2-((quinolin-4-ylmethyl)amino)ethyl)amino)carbonyl)-α-L-ribohexopyranoside;
(1-(S or R),2R,5R,6S,7S,8R,9S,11R,12S,13S)-Z-ethyl-9-hydroxy-1,5,7,9,11,13-hexamethyl-4,14-dioxo-8-((3,4,6trideoxy-3-(dimethylamino)-β-D-xylo-hexopyranosyl)oxy)-3,15-dioxabicyclo(10.2.1)pentadec-6-yl 2,6-dideoxy-3-C-methyl-3-O-methyl-4-O-(((2-((pyridin-2-ylmethyl)amino)ethyl)amino)carbonyl)-α-L-ribohexopyranoside;
(1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylohexopyranosyl)oxy)-3,15-dioxabicyclo(10.2.1)pentadec-6-yl 2,6-dideoxy-3-C-methyl-3-O-methyl-4-O-(((2-((pyridin-2-ylmethyl)amino)ethyl)amino)carbonyl)-α-L-ribohexopyranoside;
(1S,2R,4S,5R,6S,7S,8R,11R,12-(S or R),13S,14R)-13-amino-11-ethyl-4,7-dihydroxy-2,4,6,8,12,14-hexamethyl-9-oxo-10,15-dioxabicyclo(10.2.1)pentadec-5-yl 3,4,6-trideoxy-3-(dimethylamino)-β-D-xylo-hexopyranoside;
benzyl (1S,2R,4S,5R,6S,7S,8R,11R,12-(S or R),13S,14R)-11-ethyl-4,7-dihydroxy-2,4,6,8,12,14-hexamethyl-9-oxo-5-((3,4,6-trideoxy-3-(dimethylamino)-β-D-xylohexopyranosyl)oxy)-10,15-dioxabicyclo(10.2.1)pentadec-13-ylcarbamate;
(1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylohexopyranosyl)oxy)-3,15-dioxabicyclo(10.2.1)pentadec-6-yl isopropylcarbamate;
(1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylohexopyranosyl)oxy)-3,15-dioxabicyclo(10.2.1)pentadec-6-yl cyclopentylcarbamate:
(1-(S or R),2R,5R,6S,79,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylohexopyranosyl)oxy)-3,15-dioxabicyclo(10.2.1)pentedec-6-yl cyclohexylcarbamate;
(1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylohexopyranosyl)oxy)-3,15-dioxabicyclo(10.2.1)pentadec-6-yl 4-fluorobenzylcarbamate;
(1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylohexopyranosyl)oxy)-3,15-dioxabicyclo(10.2.1)pentadec-6-yl 3,5-dichlorophenylcarbamate;
(1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylohexopyranosyl)oxy)-3,15-dioxabicyclo(10.2.1)pentadec-6-yl (1S,2R)-2-phenylcyclopropylcarbamate compound with (1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl (1R,2S)-2-phenylcyclopropylcarbamate (1:1);
(1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylohexopyranosyl)oxy)-3,15-dioxabicyclo(10.2.1)pentadec-6-yl propylcarbamate;
(1S,2R,4S,5R,6S,7S,8R,11R,12-(S or R),13S,14R)-13-amino-11-ethyl-7-((3-(4-fluorophenyl)-4,5-dihydroisoxazol-5-yl)methoxy)-4-hydroxy-2,4,6,8,12,19-hexamethyl-9-oxo-10,15-dioxabicyclo(10.2.1)pentadec-5-yl 3,4,6-trideoxy-3-(dimethylamino)-β-D-xylo-hexopyranoside;
(1S,2R,4S,5R,6S,7S,8R,11R,12-(S or R),13S,14R)-13-amino-11-ethyl-4-hydroxy-2,4,6,8,12,14-hexamethyl-9-oxo-7-((3-pyridin-2-yl-4,5-dihydroisoxazol-5-yl)methoxy)-10,15-dioxabicyclo(10.2.1)pentadec-5-yl 3,4,6-trideoxy-3-(dimethylamino)-β-D-xylo-hexopyranoside;
(1-(S or R),2R,5R,6S,7S,8R,9S,11R,12S,13R,14S)-14-amino-2-ethyl-9-hydroxy-1,5,7,9,11,13-hexamethyl-9-oxo-8-((3,4,6-trideoxy-3-(dimethylamino)-β-D-xylohexopyranosyl)oxy)-3,15-dioxabicyclo(10.2.1)pentadec-6-yl pyridin-2-ylmethylcarbamate;
(1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylohexopyranosyl)oxy)-3,15-dioxabicyclo(10.2.1)pentadec-6-yl 2-pyridin-4-ylethylcarbamate;
(1S,2R,4S,5R,6S,7S,8R,11R,12-(S or R),13S,14R)-13-amino-11-ethyl-4-hydroxy-2,4,6,8,12,14-hexamethyl-9-oxo-7-propoxy-10,15-dioxabicyclo(10.2.1)pentadec-5-yl 3,4,6-trideoxy-3-(dimethylamino)-β-D-xylo-hexopyranoside;
(1S,2R,4S,5R,6S,7S,8R,11R,12-(S or R),13S,14R)-13-amino-11-ethyl-4-hydroxy-2,4,6,8,12,14-hexamethyl-9-oxo-7-((3-phenyl-4,5-dihydroisoxazol-5-yl)methoxy)-10,15-dioxabicyclo(10.2.1)pentadec-5-yl 3,4,6-trideoxy-3-(dimethylamino)-β-D-xylo-hexopyranoside;
(1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylohexopyranosyl)oxy)-3,15-dioxabicyclo(10.2.1)pentadec-6-yl (3-phenyl-4,5-dihydroisoxazol-5-yl)methylcarbamate;
(1-(S or R),2R,5R,6S,7S,6R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylohexopyranosyl)oxy)-3,15-dioxabicyclo(10.2.1)pentadec-6-yl pyridin-4-ylmethylcarbamate;
(1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylohexopyranosyl)oxy)-3,15-dioxabicyclo(10.2.1)pentadec-6-yl pyridin-3-ylmethylcarbamate;
(1-(s or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylohexopyranosyl)oxy)-3,15-dioxabicyclo(10.2.1)pentadec-6-yl pyridin-2-ylacetate;
(1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylohexopyranosyl)oxy)-3,15-dioxabicyclo(10.2.1)pentadec-6-yl 2-pyridin-3-ylethylcarbamate;
(1-(S or R),2R,5R,6S,7S,8R,9S,11R,12S,13R,14S)-14-amino-2-ethyl-9-hydroxy-1,5,7,9, 11, 13-hexamethyl-4-oxo-8-((3,4,6-trideoxy-3-(dimethylamino)-β-D-xylohexopyranosyl)oxy)-3,15-dioxabicyclo(10.2.1)pentadec-6-yl 2-pyridin-2-ylethylcarbamate;
(1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylohexopyranosyl)oxy)-3,15-dioxabicyclo(10.2.1)pentadec-6-yl 3-fluorobenzylcarbamate;
(1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylohexopyranosyl)oxy)-3,15-dioxabicyclo(10.2.1)pentadec-6-yl 2-fluorobenzylcarbamate;
(1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylohexopyranosyl)oxy)-3,15-dioxabicyclo(10.2.1)pentadec-6-yl 4-methylbenzylcarbamate;
(1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylohexopyranosyl)oxy)-3,15-dioxabicyclo(10.2.1)pentadec-6-yl 3-pyridin-3-ylpropanoate;
(1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylohexopyranosyl)oxy)-3,15-dioxabicyclo(10.2.1)pentadec-6-yl 4-methoxybenzylcarbamate; and
(1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylohexopyranosyl)oxy)-3,15-dioxabicyclo(10.2.1)pentadec-6-yl benzylcarbamate.

Compounds of this invention contain asymmetrically substituted carbon atoms in the R or S configuration, in which the terms "R" and "S" are as defined by the IUPAC 1974 Recommendations for Section E, Fundamental Stereochemistry, Pure Appl. Chem, (1976) 45, 13-10. Compounds having asymmetrically substituted carbon atoms with equal amounts of R and S configurations are racemic at those carbon atoms. Atoms with an excess of one configuration over the other are assigned the configuration which is present in the higher amount, preferably an excess of about 85%-90%, more preferably an excess of about 95%-99%, and still more preferably an excess greater than about 99%. Accordingly, this invention is meant to embrace racemic mixtures, relative and absolute stereoisomers, and mixtures of relative and absolute stereoisomers of the compounds thereof.

Compounds of this invention may also contain carbon-carbon double bonds or carbon-nitrogen double bonds in the Z or E configuration, in which the term "Z" represents the larger two substituents on the same side of a carbon-carbon or carbon-nitrogen double bond and the term "E" represents the larger two substituents on opposite sides of a carbon-carbon or carbon-nitrogen double bond. The compounds may also exist as an equilibrium mixture of Z or E configurations.

Compounds of this invention which contain -OH, -NH-, or -CO₂H moieties may have attached thereto prodrug-forming moieties. The prodrug-forming moieties are removed by metabolic processes and release the compounds having the freed hydroxyl, amino, or carboxylic acid in vivo. Prodrugs are useful for adjusting such pharmacokinetic properties of the compounds as solubility and/or hydrophobicity, absorption in the gastrointestinal tract, bioavailability, tissue penetration, and rate of clearance.

Compounds of this invention may exist as acid addition salts, basic addition salts, or zwitterions. Salts of the compounds are prepared during their isolation or following their purification. Acid addition salts of the compounds are those derived from the reaction of the compounds with an acid. For example, the acetate, adipate, alginate, bicarbonate, ci.trate, aspartate, benzoate, benzenesulfonate, bisulfate, butyrate, camphorate, camphorsufonate, digluconate, formate, fumarate, glycerophosphate, glutamate, hemisulfate, heptanoate, hexanoate, hydrochloride, hydrobromide, hydroiodide, lactobionate, lactate, maleate, mesitylenesulfonate, methanesulfonate, naphthylenesulfonate, nicotinate, oxalate, pamoate, pectinate, persulfate, phosphate, picrate, propionate, succinate, tartrate, thiocyanate, trichloroacetic, trifluoroacetic, para-toluenesulfonate, and undecanoate salts of the compounds and prodrugs thereof are contemplated as being embraced by this invention. When the compounds contain carboxylic acids, basic addition salts may be prepared therefrom by reaction with a base such as the hydroxide, carbonate, or bicarbonate of cations such as lithium, sodium, potassium, calcium, and magnesium.

Compounds of this invention may be administered with or without an excipient. Excipients include encapsulating materials or formulation additives such as absorption accelerators, antioxidants, binders, buffers, coating agents, coloring agents, diluents, disintegrating agents, emulsifiers, extenders, fillers, flavoring agents, humectants, lubricants, perfumes, preservatives, propellants, releasing agents, sterilizing agents, sweeteners, solubilizers, wetting agents, and mixtures thereof. Excipients for orally administered compounds in solid dosage forms include agar, alginic acid, aluminum hydroxide, benzyl alcohol, benzyl benzoate, 1,3-butylene glycol, castor oil, cellulose, cellulose acetate, cocoa butter, corn starch, corn oil, cottonseed oil, ethanol, ethyl acetate, ethyl carbonate, ethyl cellulose, ethyl laureate, ethyl oleate, gelatin, germ oil, glucose, glycerol, groundnut oil, isopropanol, isotonic saline, lactose, magnesium hydroxide, magnesium stearate, malt, olive oil, peanut oil, potassium phosphate salts, potato starch, propylene glycol, Ringer's solution, talc, tragacanth, water, safflower oil, sesame oil, sodium carboxymethyl cellulose, sodium lauryl sulfate, sodiumphosphate salts, soybean oil, sucrose, tetrahydrofurfuryl alcohol, and mixtures thereof. Excipients for ophthalmically and orally administered compounds in liquid dosage forms include benzyl alcohol, benzyl benzoate, 1,3-butylene glycol, castor oil, corn oil, cottonseed oil, ethanol, ethyl acetate, ethyl carbonate, fatty acid esters of sorbitan, germ oil, groundnut oil, glycerol, isopropanol, olive oil, polyethylene glycols, propylene glycol, sesame oil, tetrahydrofurfuryl alcohol, water, and mixtures thereof. Excipients for osmotically administered compounds include chlorofluorohydrocarbons, ethanol, isopropanol, water, and mixtures thereof. Excipients for parenterally administered compounds include 1,3-butanediol, castor oil, corn oil, cottonseed oil, germ oil, groundnut oil, liposomes, oleic acid, olive oil, peanut oil, Ringer's solution, safflower oil, sesame oil, soybean oil, U.S.P. or isotonic sodium chloride solution, water, and mixtures thereof. Excipients for rectally and vaginally administered compounds include cocoa butter, polyethylene glycol, wax, and mixtures thereof.

Compounds of this invention may be administered orally, ophthalmically, osmotically, parenterally (subcutaneously, intramuscularly, intrasternally, intravenously), rectally, topically, transdermally, and vaginally. Orally administered compounds in solid dosage forms may be administered as capsules, dragees, granules, pills, powders, and tablets. Ophthalmically and orally administered compounds in liquid dosage forms may be administered as elixirs, emulsions, microemulsions, solutions, suspensions, and syrups. Osmotically and topically administered compounds may be administered as creams, gels, inhalants, lotions, ointments, pastes, powders, solutions, and sprays. Parenterally administered compounds may be administered as aqueous or oleaginous solutions or aqueous or oleaginous suspensions in which the suspensions comprise crystalline, amorphous, or otherwise insoluble forms of the compounds. Rectally and vaginally administered compounds may be administered as creams, gels, lotions, ointments, and pastes.

Therapeutically effective amounts of the compounds of this invention depend on the recepient of treatment, the disorder being treated and the severity thereof, the composition comprising the compounds, the time of administration, the route of administration, the duration of treatment, the potency of the compounds, and the rate of excretion of the compounds. The daily therapeutically effective amount of the compounds administered to a patient in single or divided doses range from about 0.1 to about 200 mg/kg body weight, preferably from about 0.25 to about 100 mg/kg body weight. Single dose compositions contain these amounts of the compounds or combinations of submultiples thereof.

To determine antibacterial activity of compounds of this invention, twelve petri dishes, each containing successive aqueous dilutions of test compounds in sterilized Brain Heart Infusion agar (Difco 0418-01-5) (10 mL), were inoculated with 1:100 dilutions of the representative microorganisms in TABLE 1 using a Steers replicator block (or 1:10 dilutions for slow-growing Streptococcus strains), co-incubated at 35-37°C for 20-24 hours with a plate having no compound, and inspected visually to provide the minimum inhibitory concentration (MIC), in µg/mL, by which is meant the lowest concentration of the test compound which yielded no growth, a slight haze, or sparsely isolated colonies on the inoculums spot as compared to growth in the control plate.

**TABLE 1**

| Microorganism | Code |
|---|---|
| Staphylococcus aureus NCTC10649M | AA |
| Staphylococcus aureus A5177 | BB |
| Staphylococcus aureus PIU 2043 | CC |
| Staphylococcus aureus 1775 | DD |
| Streptococcus pyrogenes EES61 | EE |
| Streptococcus pyrogenes 930 | FF |
| Streptococcus pyrogenes PIU 2548 | GG |
| Streptococcus pneumoniae ATCC 6303 | HH |
| Streptococcus pneumoniae 5979 | JJ |
| Streptococcus pneumoniae 5649 | KK |
| Enterococcus faecalis PIU 1967 | LL |
| Enterococcus faecium GYR 1632 | MM |
| Moraxella catarrhalis 2604 | NN |
| Haemophilus influenzae GYR 1435 | PP |
| Escherichia coli JUHL | QQ |

Representative compounds of this invention displayed antibacterial activity superior to the control, which control demonstrated no antibacterial activity. This antibacterial activity demonstrates the usefulness of the compounds as antibacterials.

It is also meant to be understood that certain metabolites of compounds of this invention, which metabolites are produced by in vitro or in vivo metabolic processes, would also be useful as antibacterials and are meant to be embraced by this invention.

It is still also meant to be understood that certain precursor compounds, which precursor compounds may be metabolized in vitro or in vivo to form compounds of this invention, are meant to be embraced by this invention,

Compounds of this invention may also be prepared by synthetic chemical processes, examples of which synthetic chemical processes, and intermediates employed in the processes, are shown hereinbelow. It is meant to be understood that the order of the steps in the processes may be varied, reagents, solvents, and reaction conditions may be substituted for those specifically mentioned, and vulnerable moieties may be protected and deprotected, as necessary, during the process.

Abbreviations used herein are CBZ-NOS for N-(benzyloxycarbonyloxy)succinimide; CDI for 1,1'-carbonyldiimidazole; DBU for 1,8-diazabicyclo(5.4.0)undec-7-ened; dppe for 1,2-bis(diphenylphosphino)ethane; DIEA for N,N-diisopropylethylamine; DMAP for 4-(N,N-dimethylamino)pyridine; DMF for N,N-dimethylformamide; EDCI for 1-(3-dimethylaminopropyl)-3-carbodiimide hydrochloride; THF for tetrahydrofuran.

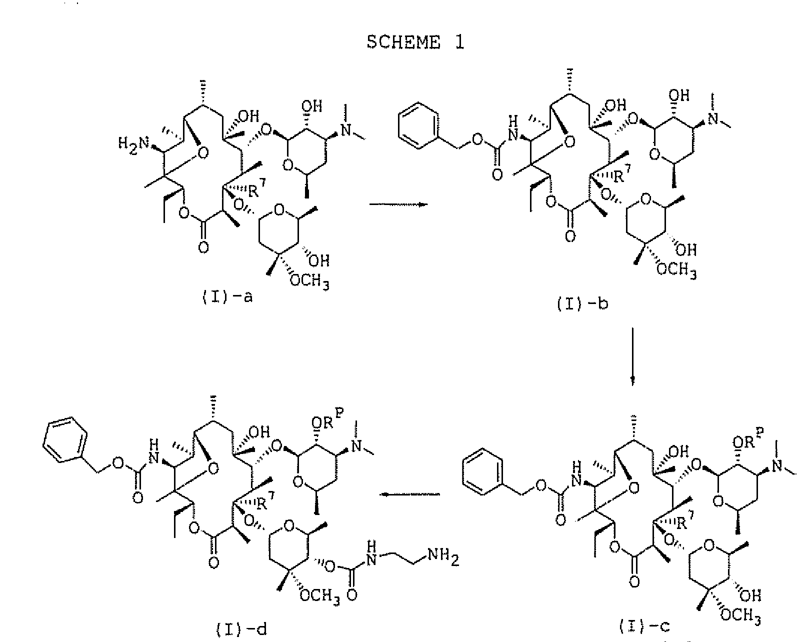

Compound having formula (I)-a may be prepared from erythromycin A as described in Bioorg, & Medicinal Chemistry Letters, Vol. 5, No. 12, 1307-1310.

Compounds having formula (I)-a may be converted to compounds having formula (I)-b by reacting the former, an amino-protecting group precursor, and a first base.

Examples of amino-protecting group precursors include benzyl chloroformate, dibenzyl dicarbonate, and N-(benzyloxycarbonyloxy)succinimide.

Examples of first bases include pyridine, sodium bicarbonate, sodium carbonate, triethylamine, tributylamine, and diisopropylethylamine.

The reaction is typically conducted over about 1 hour to about 3 days, at about -10°C to about 35°C, in solvents such as dichloromethane, methanol, tetrahydrofuran, ether, N,N-dimethylformamide, acetonitrile, ethyl acetate, acetone, 1,2-dimethoxyethane, dimethylsulfoxide, dioxane, chloroform, and mixtures thereof.

Compounds having formula (I)-b may be converted to compounds having formula (I)-c by reacting the former, a hydroxyl-protecting group precursor, and the first base, with or without 4-(N,N-dimethylamino)pyridine.

Examples of hydroxyl-protecting group precursors include acetic anhydride, benzoic anhydride, benzyl chloroformate, hexamethyldisilazane, trimethylsilyl chloride, and triethylsilyl chloride.

The reaction is typically conducted over about 1 hour to about 48 hours, at about -10°C to about 75°C, in solvents such as tetrahydrofuran, ether, N,N-dimethylformamide, acetonitrile, ethyl acetate, acetone, 1,2-dimethoxyethane, dichloromethane, chloroform, and mixtures thereof.

Compounds having formula (I)-c may be converted to compounds having formula (I)-d by (a) reacting the former, a carbonylating agent, and a second base, with or without 4-(N,N-dimethylamino)pyridine, and (b) reacting the product of step (a) and 2-aminoethylamine.

Examples of carbonylating agents include 1,1'-carbonyldiimidazole, phosgene, diphosgene, triphosgene and disuccinimidyl carbonate.

Examples of second bases include 1,8-diazabicyclo(5.4.0)undec-7-ene, triethylamine, diisopropylethyl amine, pyridine, and lutidine.

Step (a) is typically conducted at about -78°C to about 100°C, over about 1 hour to about 24 hours, in solvents such as toluene, ether, tetrahydrofuran, dichloromethane, N,N-dimethylformamids, benzene, pyridine and mixtures thereof.

Step (b) is typically conducted at about 0°C to about 50°C over about 1 hour to about 4 days in solvents such as tetrahydrofuran, acetonitrile, dichloromethane, chloroform, toluene, benzene ether, and mixtures thereof.

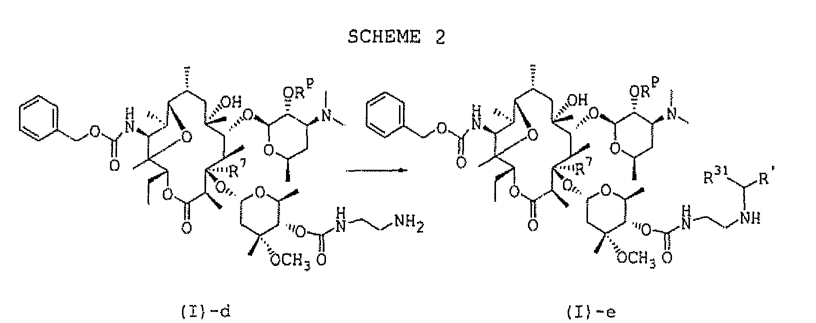

Compounds having formula (I)-d may be converted to compounds having formula (I)-e by (a) reacting the former and a compound having formula R³¹C(O)R', in which R' is hydrogen or alkyl, and (b) reacting the product of step (a) and a reducing agent, with or without a first acid.

Examples of compounds having formula R³¹C(O)R' in which R' is hydrogen include pyridine-2-carbaldehyde, pyridine-3-carbaldehyde, pyridine-4-carbaldehyde, pyrimidine-4-carbaldehyde, quinoline-3-carbaldehyde, quinoline-4-carbaldehyde, phenylacetaldehyde, 2-(trifluoromethyl)benzaldehyde, 2-methoxybenzaldehyde, and cinnamaldehyde.

Examples of compounds having formula R³¹C(O)R' in which R' is alkyl include 1-phenylethanone, 1-(3,4-dichlorophenyl)propan-1-one, 1-(2-methoxyphenyl)propan-1-one, 1-(2-methoxyphenyl)ethanone, 2,2,2-trifluoro-1-(2-methoxyphenyl)ethanone, and 1-(3,5-bis(trifluoromethyl)phenyl)ethanone.

Examples of the reducing agents include sodium borohydride, sodium cyanoborohydride, sodium triacetoxyborohydride, zinc and hydrochloric acid, iron pentacarbonyl and alcoholic potassium hydroxide, borane-pyridine, and formic acid.

Examples of first acids include acetic acid, formic acid, and hydrochloric acid.

Step (a) is typically conducted at about 25°C to about 150°C, over about 1 hour to about 24 hours, in solvents such as tetrahydrofuran, dichloromethane, toluene, benzene, dimethyl sulfoxide, acetonitrile, xylene, N,N-dimethylformamide, and mixtures thereof.

Step (b) is typically conducted at about -10°C to about 50°C, over about 1 hour to about 24 hours, in solvents such as acetonitrile, methanol, ethanol, dichloromethane, toluene, benzene, N,N-dimethylformaide, and mixtures thereof.

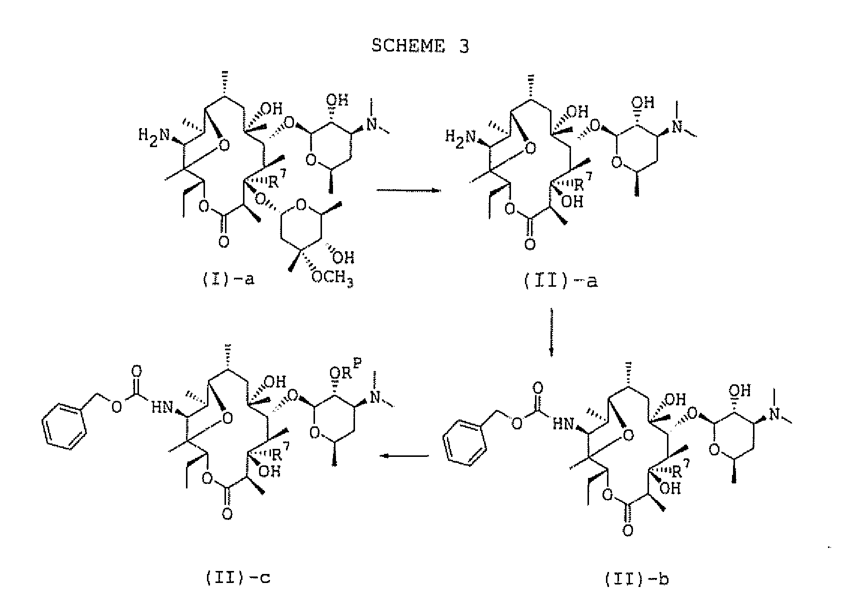

Compounds having formula (I)-a may be converted to compounds having formula (II)-a by reacting the former and a second acid.

Examples of second acids include hydrochloric acid, sulfuric acid, perchloric acid, chloroacetic acid, dichloroacetic acid, and trifluoroacetic acid.

The reaction is typically conducted at about -10°C to about 70°C, over about 1 hour to about 72 hours, in solvents such as dichloromethane, tetrahydrofuran, methanol, water, ethanol, isopropanol, butanol, and mixtures thereof.

Compounds having formula (II)-a may be converted to compounds having formula (II)-b by using the same reagents and under the same conditions described for the conversion of compounds having formula (I)-a to compounds having formula (I)-b in SCHEME 1.

Compounds having formula (II)-b may be converted to compounds having formula (II)-c by using the same reagents and under the same conditions described for the conversion of compounds having formula (I)-b to compounds having formula (I)-c in SCHEME 1.

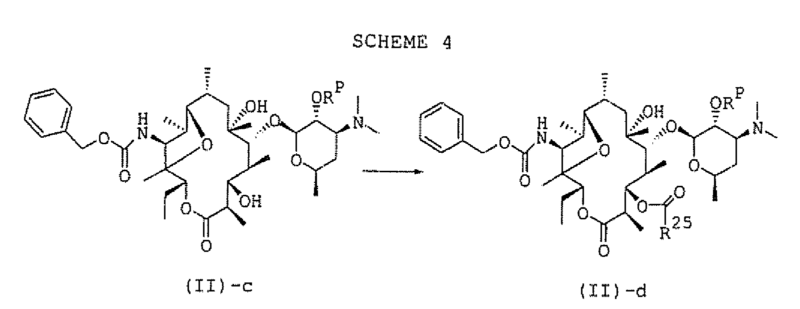

Compounds having formula (II)-c may be converted to compounds having formula (II)-d by reacting the former, a compound having formula R²⁵COOH, an acid activating agent, with or without the second base, and with or without 4-(N,N-dimethylamino)pyridine.

Examples of acid activating agents include 1-(3-dimethylaminopropyl)-3-ethylcarbodiimide hydrochloride, 1,3-dicyclohexylcarbodiimide, and thionyl chloride.

Examples of compounds having formula R²⁵COOH include 2-pyridylacetic acid, 3-pyridylpropanoic acid, phenylacetic acid, 3-quinolin-3-ylacrylic acid, 4-(5-pyridin-2-ylthien-2-yl)but-3-enoic acid, propanoic acid, and butanoic acid.

The reaction is typically conducted at about -10°C to about 35°C, over about 1 hour to about 3 days, in solvents such as dichloromethane, chloroform, toluene, ethyl acetate, acetonitrile, tetrahydrofuran, and mixtures thereof.

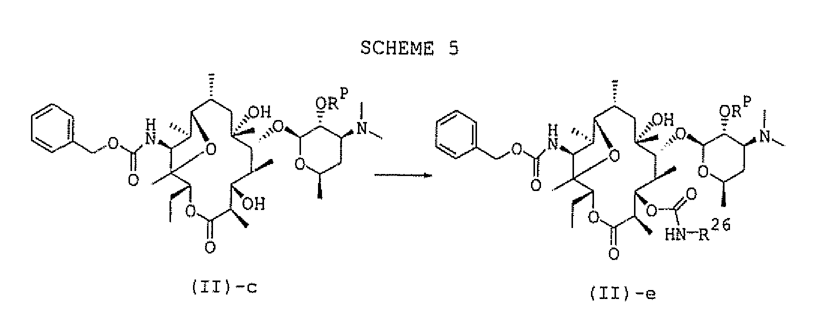

Compounds having formula (II)-c may be converted to compounds having formula (II)-e by reacting the former, a compound having formula R²⁶NCO, and 4-(N,N-dimethylamino)pyridine.

Examples of compounds having formula R²⁶NCO include ethyl isocynante, isopropyl isocyanate, allyl isocyanate, cyclopentyl isocyanate, cyclohexyl isocyanate, phenyl isocyanate, 4-fluorobenzylisocyanate, 3,5-dichlorophenyl isocyanate, trans-2-phenylcyclopropyl isocyanate, 2-methoxyphenyl isocyanate, 2-ethylphenyl isocyanate, 3,4-dichlorophenyl isocyanate, and l-naphthyl isocyanate.

The reaction is typically conducted at about 25°C to about 150°C, over about 1 hour to about 4 days, in solvents such as toluene, benzene, xylene, dichloromethane, chloroform, tetrahydrofuran, and mixtures thereof.

Alternatively, compounds having formula (II)-c may be converted to compounds having formula (II)-e by (a) reacting the former, the carbonylating agent, and the second base, with or without 4-(N,N-dimethylamino)pyridine, and (b) reacting the product of step (a) and a compound having formula H₂NR²⁶ using the same reagents and under the same conditions described for the conversion of compounds having formula (I)-c to compounds having formula (I)-d in SCHEME 1.

Examples of amines having formula H₂NR²⁶ include ethylamine, propylamine, (prop-2-ynyl)amine, (prop-2-enyl)amine, phenylmethylamine, (3-fluorophenyl)methylamine, (2-fluorophenyl)methylamine, (4-methylphenyl)methylamine, (4-methoxyphenyl)methylamine, (pyridin-2-yl)methylamine, (pyridin-3-yl)methylamine, (pyridin-4-yl)methylamine, 2-(pyridin-2-yl)ethylamine, 2-(pyridin-3-yl)ethylamine, and 2-(pyridin-4-yl)ethylamine.

Compounds having formula (I), formula (I)-f, formula (I)-g, formula (II), formula (II)-f, and formula (II)-g, in which R^{p} is acetyl or benzoyl, may be converted to compounds having formula (I), formula (I)-f, formula (I)-g, formula (II), formula (II)-f, and formula (II)-g, in which R² is hydrogen, by reacting the former and a deprotecting agent.

Examples of deprotecting agents include acids such as methanol, ethanol, acetic acid, and formic acid and bases such as lithium hydroxide, sodium hydroxide, potassium hydroxide, potassium carbonate, and ammonia.

The reaction is typically conducted at about 25°C to about 70°C, over about 1 hour to about 72 hours, in solvents such as water, methanol, ethanol, and mixtures thereof.

Compound having formula (I) or (II), in which R³ or R⁴ is (phenylmethyl)OC(O)NH-, may be converted to compounds having formula (I) or (II), in which R³ or R⁴ is -NH₂, by reacting the former, a hydride source and a palladium catalyst.

Examples of hydride sources include cyclohexene, 1,4-cyclohexadiene, formic acid, hydrogen, and ammonium formate.

Examples of palladium catalysts include palladium black, palladium on carbon, and palladium hydroxide.

The reaction is typically conducted at about 25°C to about 70°C, over about 2 hours to about 3 days, in solvents such as ethanol, isopropanol, ethyl acetate, and mixtures thereof.

The compounds and processes of this invention will be better understood in connection with the following examples.

### EXAMPLE 1

This example was prepared as described in column 34, lines 34-41 of commonly-owned US Patent No. 5,288,709.

### EXAMPLE 2

A solution of EXAMPLE 1 (14.9g) in dichloromethane (100 mL) at 0°C was treated with benzoic anhydride (6.79g) and triethylamine (4.17 mL), stirred at 25°C for 17 hours, and concentrated; and the concentrate was flash chromatographed on silica gel with 97:3:0.5 dichloromethane/methanol/ammonium hydroxide.

### EXAMPLE 3

A solution of EXAMPLE 2 (820 mg), CDI (405 mg), DMAP (12.2 mg), and DBU (224 µL) in THF (10 mL) and DMF (3 mL) at 25°C was stirred for 18 hours, treated with ethyl acetate, washed with water and saturated NaHCO₃, and dried (Na₂SO₄), filtered, and concentrated.

### EXAMPLE 4

A solution of the EXAMPLE 3 concentrate and ethylenediamine (667 µL) in acetonitrile (10 mL) and water (1 mL) at 25°C was stirred for 4 days and concentrated; and the concentrate was flash chromatographed on silica gel with 95:5:0.5 dichloromethane/methanol/ammonium hydroxide.

### EXAMPLE 5

### (1-(S or R),2R,5R,6S,7S,8R,9S,11R,12S,13S)-2-ethyl-9-hydroxy-1,5,7,9,11,13-hexamethyl-4,14-dioxo-8-((3,4,6-trideoxy-3-(dimethylamino)-β-D-xylo-hexopyranosyl)oxy)-3,15-dioxabicyclo(10.2.1)pentadec-6-yl 4-O-(((2-aminoethyl)amino)carbonyl)-2,6-dideoxy-3-C-methyl-3-O-methyl-α-L-ribo-hexopyranoside

This example was prepared by substituting EXAMPLE 4 for EXAMPLE 7 in EXAMPLE 8.

### EXAMPLE 6

A solution of EXAMPLE 3 (286 mg) and 1-(2-methoxyphenyl)ethanone (65 µL), in acetonitrile (2 mL) at 80°C was stirred for 18 hours and concentrated.

### EXAMPLE 7

A solution of the EXAMPLE 6 concentrate in methanol (2 mL) at 0°C was treated with sodium cyanoborohydride (30 mg), stirred for 3 hours, acidified to pH 3 with 1M HCl, stirred for 15 minutes at 25°C, treated with 5% Na₂CO₃ until basic, and extracted with dichloromethane; and the extract was dried (Na₂SO₄), filtered, and concentrated.

### EXAMPLE 8

### (1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy-3,15-dioxabicyclo(10.2.1)pentadec-6-yl 2,6-dideoxy-4-O-(((2-((1-(2-methoxyphenyl)ethyl)amino)ethyl)amino)carbonyl)-3-C-methyl-3-O-methyl-α-L-ribo-hexopyranoside

A solution of the EXAMPLE 7 concentrate in methanol (5 mL) was refluxed for 4 hours and concentrated; and the concentrate was flash chromatographed on silica gel with 95:5:0.5 dichloromethane/methanol/ammonium hydroxide.

### EXAMPLE 9

This example was prepared by substituting quinoline-3-carboxaldehyde for 1-(2-methoxyphenyl)ethanone in EXAMPLE 6.

### EXAMPLE 10

This example was prepared by substituting EXAMPLE 9 for in EXAMPLE 6 in EXAMPLE 7.

### EXAMPLE 11

### (1-(S or R),2R,5R,6S,7S,8R,9S,11R,12S,13S)-2-ethyl-9-hydroxy-1,5,7,9,11,13-hexamethyl-4,14-dioxo-8-((3,4,6-trideoxy-3-(dimethylamino)-β-D-xylo-hexopyranosyl)oxy)-3,15-dioxabicyclo(10.2.1)pentadec-6-yl 2,6-dideoxy-3-C-methyl-3-O-methyl-4-O-(((2-((quinolin-3-ylmethyl)amino)ethyl)amino)carbonyl)-α-L-ribohexopyranoside

This example was prepared by substituting EXAMPLE 10 for EXAMPLE 7 in EXAMPLE 8.

### EXAMPLE 12

This example was prepared by substituting quinoline-4-carboxaldehyde for 1-(2-methoxyphenyl)ethanone in EXAMPLE 6.

### EXAMPLE 13

This example was prepared by substituting EXAMPLE 12 for in EXAMPLE 6 in EXAMPLE 7.

### EXAMPLE 14

### (1-(S or R),2R,5R,6S,7S,8R,9S,11R,12S,13S)-2-ethyl-9-hydroxy-1,5,7,9,11,13-hexamethyl-4,14-dioxo-8-((3,4,6-trideoxy-3-(dimethylamino)-β-D-xylo-hexopyranosyl)oxy)-3,15-dioxabicyclo(10.2.1)pentadec-6-yl 2,6-dideoxy-3-C-methyl-3-O-methyl-4-O-(((2-((quinolin-4-ylmethyl)amino)ethyl)amino)carbonyl)-α-L-ribo-hexopyranoside

This example was prepared by substituting EXAMPLE 10 for EXAMPLE 7 in EXAMPLE 8.

### EXAMPLE 15

This example was prepared by substituting pyridine-2-carboxaldehyde for 1-(2-methoxyphenyl)ethanone in EXAMPLE 6.

### EXAMPLE 16

This example was prepared by substituting EXAMPLE 15 for in EXAMPLE 6 in EXAMPLE 7.

### EXAMPLE 17

### (1-(S or R),2R,5R,6S,7S,8R,9S,11R,12S,13S)-2-ethyl-9-hydroxy-1,5,7,9,11,13-hexamethyl-4,14-dioxo-8-((3,4,6-trideoxy-3-(dimethylamino)-β-D-xylo-hexopyranosyl)oxy)-3,15-dioxabicyclo(10.2.1)pentadec-6-yl 2,6-dideoxy-3-C-methyl-3-O-methyl-4-O-(((2-((pyridin-2-ylmethyl)amino)ethyl)amino)carbonyl)-α-L-ribo-hexopyranoside

This example was prepared by substituting EXAMPLE 16 for EXAMPLE 7 in EXAMPLE 8.

### EXAMPLE 18

A solution of EXAMPLE 1 (6.4g), hydroxylamine hydrochloride (7.33g), and triethylamine (8.62 mL) in ethanol (80 mL) at 70°C was stirred for four days, cooled, and concentrated. The concentrate was dissolved in 5% NaHCO₃, adjusted to pH 10 with concentrated ammonium hydroxide, and extracted with dichloromethane. The extract was washed with water and 10% NaHCO₃ and dried (Na₂SO₄), filtered, and concentrated; and the concentrate was purified on silica gel with 97:3:0.5 to 95:5:0.5 dichloromethane/methanol/ammonium hydroxide.

### EXAMPLE 19

A solution of EXAMPLE 18 (4.48g) and ammonium acetate (23.51g) in methanol (100 mL) at 0°C was treated with 30% TiCl₃ in 2M HCl (5.23 mL), stirred for 1 hour, treated with sodium cyanoborohydride (1.92g), stirred for 18 hours at 25°C, cooled to 0°C, treated with additional 30% TiCl₃ in 2M HCl (5.2 mL), stirred for another 18 hours at 25°C, and concentrated.

### EXAMPLE 20

A solution of EXAMPLE 19 (3.93g) and triethylamine (2.29 mL) in dichloromethane (50 mL) at 25°C was treated with di-tert-butyl dicarbonate (1.32g) in dichloromethane (10 mL), stirred for 1 hour at room temperature, diluted with dichloromethane, washed with water and saturated NaHCO₃, and dried (Na₂SO₄), filtered, and concentrated; and the concentrate was purified on silica gel with 97:3:0.5 dichloromethane/methanol/ammonium hydroxide.

### EXAMPLE 21

A solution of EXAMPLE 20 (1.9g) in dichloromethane (10 mL) at 0°C was treated with benzoic anhydride (789 mg) and triethylamine (485 L), stirred at 25°C for 17 hours, and concentrated; and the concentrate was flash chromatographed on silica gel with 97:3:0.5 dichloromethane/methanol/ammonium hydroxide.

### EXAMPLE 22

A solution of EXAMPLE 21 (185 mg), CDI (81 mg), DMAP (2.4 mg), and DBU (45 µL) in THF (2 mL) and DMF (0.6 mL) at 25°C was stirred for 18 hours, treated with ethyl acetate, washed with water and saturated NaHCO₃, and dried (Na₂SO₄), filtered, and concentrated.

### EXAMPLE 23

A solution of the EXAMPLE 22 concentrate and ethylenediamine (133 µL) in acetonitrile (10 mL) and water (1 mL) at 25°C was stirred for 2 days and concentrated; and the concentrate was flash chromatographed on silica gel with 95:5:0.5 dichloromethane/methanol/ammonium hydroxide.

### EXAMPLE 24

A solution of EXAMPLE 23 (182 mg) and pyridine-2-carboxaldehyde (29 µL) in acetonitrile (2 mL) at 80°C was stirred for 18 hours and concentrated.

### EXAMPLE 25

A solution of the EXAMPLE 24 concentrate in methanol (2 mL) at 0°C was treated with sodium cyanoborohydride (19 mg), stirred for 3 hours, acidified to pH 3 with 1M HCl, stirred for 15 minutes at 25°C, made basic with 5% Na₂CO₃, and extracted with dichloromethane. The extract was dried (Na₂SO₄), filtered, and concentrated; and the concentrate was flash chromatographed on silica gel with 98:2:0.5 dichloromethane/methanol/ammonium hydroxide.

### EXAMPLE 26

### (1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl 2,6-dideoxy-3-C-methyl-3-O-methyl-4-O-(((2-((pyridin-2-ylmethyl)amino)ethyl)amino)carbonyl)-α-L-ribohexopyranoside

A solution of EXAMPLE 25 (1.98g) in methanol (5 mL), was refluxed for 4 hours and concentrated; and the concentrate was flash chromatographed on silica gel with 95:5:0.5 dichloromethane/methanol/ammonium hydroxide.

### EXAMPLE 27

### (1S,2R,4S,5R,6S,7S,8R,11R,12-(S or R),13S,14R)-13-amino-11-ethyl-4,7-dihydroxy-2,4,6,8,12,14-hexamethyl-9-oxo-10,15-dioxabicyclo(10.2.1)pentadec-5-yl 3,4,6-trideoxy-3-(dimethylamino)-β-D-xylo-hexopyranoside

A solution of EXAMPLE 19 (3g) and 1M HCl (15 mL) in ethanol (3 mL) and water (7 mL) was stirred at 25°C for 36 hours, poured into 5% NaHCO₃ (100 mL) , and extracted with chloroform; and the extract was dried (MgSO₄), filtered, and concentrated.

### EXAMPLE 28

### benzyl (1S,2R,4S,5R,6S,7S,8R,11R,12-(S or R),13S,14R)-11-ethyl-4,7-dihydroxy-2,4,6,8,12,14-hexamethyl-9-oxo-5-((3,4,6-trideoxy-3-(dimethylamino)-β-D-xylohexopyranosyl)oxy)-10,15-dioxabicyclo(10.2.1)pentadec-13-ylcarbamate

A solution of EXAMPLE 27 (63.3g) and CBZ-NOS (28.2g) in dichloromethane (1L) was stirred at 25°C for 48 hours, washed with 0.5M NaOH (100 mL), and dried (MgSO₄), filtered, and concentrated.

### EXAMPLE 29

A solution of EXAMPLE 28 (78g), triethylamine (22.8g) and acetyl chloride (28.8g) in dichloromethane (1 L) was stirred for 12 hours, washed with saturated NaHCO₃ and brine, and dried (MgSO₄), filtered, and concentrated; and the concentrate was flash chromatographed on silica gel with 97.5:2:0.5 dichloromethane/methanol/ammonium hydroxide.

### EXAMPLE 30

A solution of EXAMPLE 29 (367 mg), isopropylisocyanate (64 µL), and DMAP (61 mg) in toluene (5 mL) was heated at 100°C for 3 days and concentrated; and the concentrate was flash chromatographed on silica gel with 1:1 acetone/hexane.

### EXAMPLE 31

### (1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl isopropylcarbamate

A solution of EXAMPLE 30, 10% palladium on carbon (50 mg), and ammonium formate (315 mg) in methanol (5 mL) was heated at reflux for 6 hours and cooled, filtered through diatomaceous earth (Celite®), and concentrated; and the concentrate was flash chromatographed on silica gel with 95:4.5:0.5 dichloromethane/methanol/ammonium hydroxide.

### EXAMPLE 32

This example was prepared by substituting cyclopentylisocyanate for isopropylisocyanate in EXAMPLE 30.

### EXAMPLE 33

### (1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl cyclopentylcarbamate

This example was prepared by substituting EXAMPLE 32 for EXAMPLE 30 in EXAMPLE 31.

### EXAMPLE 34

This example was prepared by substituting cyclohexylisocyanate for isopropylisocyanate in EXAMPLE 30.

### EXAMPLE 35

### (1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl cyclohexylcarbamate

This example was prepared by substituting EXAMPLE 34 for EXAMPLE 30 in EXAMPLE 31.

### EXAMPLE 36

This example was prepared by substituting 4-fluorobenzylisocyanate for isopropylisocyanate in EXAMPLE 30.

### EXAMPLE 37

### (1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl 4-fluorobenzylcarbamate

This example was prepared by substituting EXAMPLE 36 for EXAMPLE 30 in EXAMPLE 31.

### EXAMPLE 38

This example was prepared by substituting 3,5-dichlorophenylisocyanate for isopropylisocyanate in EXAMPLE 30.

### EXAMPLE 39

### (1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl 3,5-dichlorophenylcarbamate

This example was prepared by substituting EXAMPLE 38 for EXAMPLE 30 in EXAMPLE 31.

### EXAMPLE 40 and EXAMPLE 41

These examples were prepared by substituting trans-phenylcyclopropylisocyanate for isopropylisocyanate in EXAMPLE 30.

### EXAMPLE 42 and EXAMPLE 43

### (1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl (1S,2R)-2-phenylcyclopropylcarbamate and (1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl (1R,2S)-2-phenylcyclopropylcarbamate (1:1)

These examples were prepared by substituting EXAMPLE 40 and EXAMPLE 41 for EXAMPLE 30 in EXAMPLE 31.

### EXAMPLE 44

This example was prepared by substituting allylisocyanate for isopropylisocyanate in EXAMPLE 30.

### EXAMPLE 45

### (1-(S or R),2R,5R,6S,3S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl propylcarbamate

This example was prepared by substituting EXAMPLE 44 for EXAMPLE 30 in EXAMPLE 31.

### EXAMPLE 46

A suspension of tris(dibenzylideneacetone)dipalladium (0) (4 mg) and dppe (4 mg) in THF (15 mL) was treated with EXAMPLE 30 (500 mg) and tert-butyl allyl carbonate (70 mg), refluxed for 5 hours, treated with more tris(dibenzylideneacetone)dipalladium(0) (4 mg) and tert-loutyl allyl carbonate (35g), refluxed for another 5 hours and cooled, treated with ethyl acetate, washed with saturated NaHCO₃, water, and brine, and dried (MgSO₄), filtered, and concentrated; and the concentrate was flash chromatographed on silica gel with 85:15:1.5 hexanes/acetone/triethylamine.

### EXAMPLE 47

A solution of 4-fluorophenyl oxime (400g) N-chlorosuccinamide (380 mg) and pyridine (catalytic) in dichloromethane (20 mL) was stirred for 5 hours, added to a solution of EXAMPLE 46 (230 mg) in dichloromethane (5 mL), treated with triethylamine (300 mg), stirred for 12 hours, treated with ethyl acetate, washed with saturated NaHCO₃, water, and brine, and dried (MgSO₄), filtered, and concentrated; and the concentrate was flash chromatographed on silica gel with 70:30:1.5 hexane/acetone/triethylamine.

### EXAMPLE 48

### (13,2R,4S,5R,6S,7S,8R,11R,12-(S or R),13S,14R)-13-amino-11-ethyl-7-((3-(4-fluorophenyl)-4,5-dihydroisoxazol-5-yl)methoxy)-4-hydroxy-2,4,6,8,12,14-hexamethyl-9-oxo-10,15-dioxabicyclo(10.2.1)pentadec-5-yl 3,4,6-trideoxy-3-(dimethylamino)-β-D-xylo-hexopyranoside

A solution of EXAMPLE 47 (220 mg) and 10% palladium on carbon (30 mg) in methanol (10 mL) was stirred under hydrogen at 25°C for 12 hours, filtered through diatomaceous earth (Celite®), and concentrated; and the concentrate was flash chromatographed on silica gel with 97.5:2.5:1 to 95:5:1 dichloromethane/methanol/ammonium hydroxide.

### EXAMPLE 49

This example was prepared by substituting 2-pyridyl oxime for 4-fluorophenyl oxime in EXAMPLE 47.

### EXAMPLE 50

### (1S,2R,4S,5R,6S,7S,8R,11R,12-(S or R),13S,14R)-13-amino-11-ethyl-4-hydroxy-2,4,6,8,12,14-hexamethyl-9-oxo-7-((3-pyridin-2-yl-4,5-dihydroisoxazol-5-yl)methoxy)-10,15-dioxabicyclo(10.2.1)pentadec-5-yl 3,4,6-trideoxy-3-(dimethylamino)-β-D-xylo-hexopyranoside

This example was prepared by substituting EXAMPLE 49 for EXAMPLE 47 in EXAMPLE 48.

### EXAMPLE 51

A solution of EXAMPLE 29 (5g) and CDI (2.2g) in 1:1 dichloromethane/THF (100 mL) was heated at reflux for 12 hours, treated with ethyl acetate, washed with saturated NaHCO₃, water, and brine, and dried (MgSO₄), filtered, and concentrated; and the concentrate was purified by flash chromatography on silica gel with 70:30:1.5 hexane/acetone/triethylamine.

### EXAMPLE 52

A solution of EXAMPLE 51 (200g) and 2-(aminomethyl)pyridine (400g) in 5:1 acetonitrile/water (6 mL) was stirred for 12 hours, treated with ethyl acetate, washed with saturated NaHCO₃, water, and brine, and dried (MgSO₄), filtered, and concentrated; and the concentrate was flash chromatographed on silica gel with 50:50:1.5 to 70:30:1.5 acetone/hexanes/triethylamine.

### EXAMPLE 53

### (1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl pyridin-2-ylmethylcarbamate

A solution of EXAMPLE 52 and 10% palladium on carbon in methanol (10 mL) at 25°C was stirred under hydrogen for 12 hours, filtered through diatomaceous earth (Celite®), and concentrated.

### EXAMPLE 54

This example was prepared by substituting 2-(2-aminoethyl)pyridine for 2-(aminomethyl)pyridine in EXAMPLE 52.

### EXAMPLE 55

### (1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl 2-pyridin-4-ylethylcarbamate

This example was prepared by substituting EXAMPLE 54 for EXAMPLE 52 in EXAMPLE 53.

### EXAMPLE 56

### (1S,2R,4S,5R,6S,7S,8R,11R,12-(S or R),13S,14R)-13-amino-11-ethyl-4-hydroxy-2,4,6,8,12,14-hexamethyl-9-oxo-7-propoxy-10,15-dioxabicyclo(10.2.1)pentadec-5-yl 3,4,6-trideoxy-3-(dimethylamino)-β-D-xylo-hexopyranoside

A suspension of EXAMPLE 46 (200 mg) and 10% palladium on carbon (20 mg) in methanol (10 mL) at 25°C was stirred for 12 hours, filtered through diatomaceous earth (Celite®), and concentrated.

### EXAMPLE 57

This example was prepared by substituting phenyl oxime for 4-fluorophenyl oxime in EXAMPLE 47.

### EXAMPLE 58

### (1S,2R,4S,5R,6S,7S,8R,11R,12-(S or R),13S,14R)-13-amino-11-ethyl-4-hydroxy-2,4,6,8,12,14-hexamethyl-9-oxo-7-((3-phenyl-4,5-dihydroisoxazol-5-yl)methoxy)-10,15-dioxabicyclo(10.2.1)pentadec-5-yl 3,4,6-trideoxy-3-(dimethylamino)-β-D-xylo-hexopyranoside

This example was prepared by substituting EXAMPLE 57 for EXAMPLE 52 in EXAMPLE 53.

### EXAMPLE 59

This example was prepared by substituting EXAMPLE 44 and phenyl oxime for EXAMPLE 46 and 4-fluorophenyl oxime, respectively, in EXAMPLE 47.

### EXAMPLE 60

### (1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl (3-phenyl-4,5-dihydroisoxazol-5-yl)methylcarbamate

This example was prepared by substituting EXAMPLE 59 for EXAMPLE 52 in EXAMPLE 53.

### EXAMPLE 61

This example was prepared by substituting 4-(aminomethyl)pyridine for 2-(aminomethyl)pyridine in EXAMPLE 52.

### EXAMPLE 62

### (1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl pyridin-4-ylmethylcarbamate

This example was prepared by substituting EXAMPLE 61 for EXAMPLE 52 in EXAMPLE 53.

### EXAMPLE 63

This example was prepared by substituting 3-(aminomethyl)pyridine for 2-(aminomethyl)pyridine in EXAMPLE 52.

### EXAMPLE 64

### (1-(S or R),2R,5R,6S,7S,8R,9S,11,R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl pyridin-3-ylmethylcarbamate

This example was prepared by substituting EXAMPLE 63 for EXAMPLE 52 in EXAMPLE 53.

### EXAMPLE 65

A solution of EXAMPLE 30 (0.5g), 2-pyridylacetic acid (180 mg), DIEA (120 mg), EDCI (200 mg), and DMAP (catalytic) in dichloromethane (20 mL) was stirred for 12 hours, diluted with ethyl acetate, washed with saturated NaHCO₃, water, and brine, and dried (MgSO₄), filtered, and concentrated.

### EXAMPLE 66

### (1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl pyridin-2-ylacetate;

A solution of EXAMPLE 65 and 10% palladium on carbon (50 mg) in methanol (10 mL) at 25°C was stirred under hydrogen at 25°C for 12 hours, filtered through diatomaceous earth (Celite®), and concentrated; and the concentrate was flash chromatographed on silica gel with 95:5:1 dichloromethane/methanol/ammonium hydroxide.

### EXAMPLE 67

This example was prepared by substituting 3-(2-aminoethyl)pyridine for 2-(aminomethyl)pyridine in EXAMPLE 52.

### EXAMPLE 68

### (1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl 2-pyridin-3-ylethylcarbamate

This example was prepared by substituting EXAMPLE 67 for EXAMPLE 65 in EXAMPLE 66.

### EXAMPLE 69

This example was prepared by substituting 2-(2-aminoethyl)pyridine for 2-(aminomethyl)pyridine in EXAMPLE 52.

### EXAMPLE 70

### (1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl 2-pyridin-2-ylethylcarbamate

This example was prepared by substituting EXAMPLE 69 for EXAMPLE 65 in EXAMPLE 66.

### EXAMPLE 71

This example was prepared by substituting 3-fluorobenzylisocyanate for isopropylisocyanate in EXAMPLE 30.

### EXAMPLE 72

### (1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl 3-fluorobenzylcarbamate

This example was prepared by substituting EXAMPLE 71 for EXAMPLE 65 in EXAMPLE 66.

### EXAMPLE 73

This example was prepared by substituting 2-fluorobenzylisocyanate for isopropylisocyanate in EXAMPLE 30.

### EXAMPLE 74

### (1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl 2-fluorobenzylcarbamate

This example was prepared by substituting EXAMPLE 73 for EXAMPLE 65 in EXAMPLE 66.

### EXAMPLE 75

This example was prepared by substituting 4-methylbenzylisocyanate for isopropylisocyanate in EXAMPLE 30.

### EXAMPLE 76

### (1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl 4-methylbenzylcarbamate

This example was prepared by substituting EXAMPLE 75 for EXAMPLE 65 in EXAMPLE 66.

### EXAMPLE 77

This example was prepared by substituting 3-pyridylacetic acid for 2-pydidylacetic acid in EXAMPLE 65.

### EXAMPLE 78

### (1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl 3-pyridin-3-ylpropanoate

This example was prepared by substituting EXAMPLE 77 for EXAMPLE 65 in EXAMPLE 66.

### EXAMPLE 79

This example was prepared by substituting 4-methoxybenzylisocyanate for isopropylisocyanate in EXAMPLE 4

### EXAMPLE 80

### (1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl 4-methoxybenzylcarbamate

This example was prepared by substituting EXAMPLE 79 for EXAMPLE 65 in EXAMPLE 66.

### EXAMPLE 81

This example was prepared by substituting benzylisocyanate for isopropylisocyanate in EXAMPLE 4.

### EXAMPLE 82

### (1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl benzylcarbamate

This example was prepared by substituting EXAMPLE 81 for EXAMPLE 65 in EXAMPLE 66.

### SPECTRAL DATA

### EXAMPLE 5

¹³C NMR (CDCl₃) δ 217.6, 177.0, 157.1, 105.1, 98.2, 84.0, 82.7, 80.1, 80.0, 78.7, 78.3, 73.7, 73.6, 73.2, 70.7, 69.1, 64.6, 63.5, 55.2, 52.3, 48.9, 47.8, 47.7, 47.6, 46.6, 43.5, 42.9, 41.2, 40.4, 36.8, 35.5, 29.4, 26.6, 22.5, 22.2, 21.2, 21.0, 20.9, 19.9, 19.4, 17.2, 15.8, 13.5, 10.8, 10.3.

### EXAMPLE 8

NMR (CDCl₃) δ 217.6, 177.0, 157.1, 156.4, 132.9, 128.1, 127.6, 126.9, 120.7, 110.6, 105.2, 105.1, 98.2, 84.0, 82.7, 80.1, 80.0, 78.7, 78.3, 73.7, 73.6, 73.2, 70.7, 69.1, 64.6, 63.5, 55.2, 52.3, 49.7, 47.7, 47.6, 46.6, 43.5, 42.9, 41.2, 40.4, 36.8, 35.5, 29.4, 26.6, 22.5, 22.2, 21.2, 21.0, 20.9, 19.9, 19.4, 17.2, 15.8, 13.5, 10.8, 10.3.

### EXAMPLE 11

¹³C NMR (CDCl₃) δ 217.6, 177.0, 156.4, 151.3, 147.5, 138.9, 134.4, 132.6, 129.2, 129.1, 128.1, 127.9, 127.5, 126.7, 105.3, 98.2, 87.9, 86.9, 84.0, 93.2, 82.8, 80.3, 79.5, 78.8, 78.3, 73.6, 73.2, 70.7, 70.4, 69.3, 69.1, 64.6, 63.5, 62.8, 51.1, 49.7, 49.6, 49.1, 48.7, 47.8, 46.1, 43.3, 42.8, 40.9, 40.3, 36.9, 35.4, 30.8, 29.6, 29.4, 26.6, 24.3, 23.2, 22.5, 21.2, 20.9, 19.9, 19.4, 17.2, 16.8, 15.9, 13.9, 13.5, 12.1, 11.0, 10.8, 10.3.

### EXAMPLE 14

¹³C NMR (CDCl₃) δ 217.6, 176.9, 156.4, 150.2, 148.2, 145.2, 130.2, 129.1, 128.1, 126.9, 126.6, 123.1, 119.7, 105.3, 98.2, 88.8, 88.0, 86.9, 84.0, 83.2, 82.8, 80.3, 79.5, 78.9, 78.4, 73.5, 73.2, 70.7, 69.3, 69.1, 65.3, 64.5, 63.5, 62.8, 49.7, 49.2, 47.8, 43.5, 43.3, 42.8, 40.9, 40.3, 37.0, 35.4, 30.8, 29.6, 29.4, 26.5, 23.2, 22.5, 21.1, 20.9, 19.8, 19.4, 17.2, 16.0, 13.9, 13.5, 12.1, 10.8, 10.3.

### EXAMPLE 17

¹³C NMR (CDCl₃) δ 217.6, 177.0, 159.4, 156.4, 149.3, 138.9, 136.4, 128.2, 122.2, 122.0, 105.3, 105.1, 98.2, 88.8, 87.7, 86.9, 84.0, 82.8, 80.1, 78.7, 78.3, 73.6, 73.2, 70.7, 70.4, 69.3, 69.1, 63.6, 62.8, 54.6, 49.7, 49.1, 48.5, 47.7, 46.1, 43.5, 42.8, 40.9, 40.4, 36.9, 35.5, 30.8, 29.6, 29.4, 26.5, 24.3, 23.2, 22.6, 21.2, 20.9, 19.9, 19.4, 17.2, 16.8, 15.8, 15.6, 13.5, 12.1, 11.0, 10.8, 10.3.

### EXAMPLE 26

¹H NMR (CDCl₃) δ 8.64 (m, 1H), 7.80 (m, 1H) , 7.25 (m, 1H), 7.17 (m, 1H), 5.46 (m, 1H), 5.09 (m, 1H), 4.88 (m, 1H), 4.56 (m, 1H), 4.43-4.36 (m, 1H), 3.89 (m, 1H), 3.60-3.15 (m, 11H), 2.80 (m, 2H), 2.56 (m, 1H), 2.45-2.05 (m, 9H), 1.70-1.48 (m, 11H), 1.48-1.24 (m, 3H), 1.18-1.06 (m, 11H), 1.02 (m, 6H), 0.85 (m, 6H).

### EXAMPLE 27

¹³C NMR (CDCl₃) δ 176.2, 106.1, 93.9, 88.0, 83.6, 78.5, 76.5, 74.2, 70.4, 69.8, 65.3, 55.4, 45.0, 40.2, 38.2, 36.8, 31.4, 28.0, 27.6, 23.6, 23.0, 21.0, 18.0, 15.2, 14.8, 11.0, 7.8.

### EXAMPLE 28

¹³C NMR (CDCl₃) δ 176.5, 155.7, 136.3, 128.5, 128.2, 128.0, 106.1, 94.6, 88.6, 83.1, 78.5, 76.2, 74.2, 70.4, 69.6, 67.0, 65.2, 55.6, 45.2, 40.2, 38.6, 36.6, 32.3, 28.0, 27.8, 23.2, 23.0, 21.0, 19.6, 15.4, 14.6, 10.7, 7.6.

### EXAMPLE 33

¹³C NMR (CDCl₃) δ 174.3, 155.6, 104.1, 86.6, 85.5, 84.4, 78.6, 77.4, 73.9, 70.5, 69.4, 65.9, 54.4, 44.1, 40.2, 37.2, 36.1, 34.4, 33.6, 33.4, 28.6, 28.5, 25.3, 24.1, 23.7, 23.5, 21.1, 20.5, 16.8, 15.5, 14.4, 10.8, 9.4.

### EXAMPLE 35

¹³C NMR (CDCl₃) δ 174.2, 155.4, 104.1, 86.5, 85.7, 84.3, 78.3, 77.4, 73.9, 70.5, 69.3, 65.8, 54.4, 49.6, 44.1, 40.2, 37.2, 36.1, 34.4, 33.8, 33.6, 33.3, 28.7, 28.5, 25.4, 25.3, 24.7, 24.6, 24.0, 21.1, 20.5, 16.8, 15.4, 14.5, 10.9, 9.4.

### EXAMPLE 37

¹³C NMR (CDCl₃) δ 174.3, 163.5, 156.3, 134.8, 129.3, 129.2, 115.3, 115.1, 103.6, 86.4, 85.1, 84.4, 78.9, 77.4, 74.1, 74.0, 70.5, 69.1, 65.2, 54.2, 43.9, 40.1, 37.0, 36.0, 34.1, 28.7, 28.9, 25.1, 24.0, 20.8, 20.4, 16.7, 15.2, 14.4, 10.8, 9.3.

### EXAMPLE 39

¹³C NMR (CDCl₃) δ 174.0, 153.1, 138.2, 128.9, 123.0, 118.2, 104.1, 86.6, 85.7, 84.3, 79.5, 77.5, 74.1, 70.4, 69.3, 65.7, 54.4, 47.5, 43.7, 40.1, 37.2, 36.1, 34.3, 28.6, 28.3, 25.3, 24.0, 20.9, 20.5, 16.8, 15.5, 14.5, 10.9, 9.4.

### EXAMPLE 42 and EXAMPLE 43

¹³C NMR (CDCl₃) δ two sets of peaks at 174.2, 155.3, 137.9, 129.4, 128.3, 126.4, 104.0, 86.5, 85.3, 84.3, 78.4, 77.4, 73.9, 70.5, 69.4, 65.8, 54.4, 47.9, 44.2, 42.9, 40.2, 37.2, 36.1, 34.3, 28.6, 25.3, 24.0, 21.1, 20.5, 16.8, 15.4, 14.7, 10.8, 9.5.

### EXAMPLE 45

¹³C NMR (CDCl₃) δ 174.2, 156.2, 104.2, 86.6, 85.6, 84.4, 78.6, 77.4, 74.0, 70.5, 69.4, 65.9, 54.5, 44.2, 42.7, 40.3, 37.3, 36.2, 34.4, 29.7, 28.8, 28.6, 25.4, 24.1, 23.3, 21.1, 20.6, 15.9, 15.5, 14.6, 11.3, 10.9, 9.5.

### EXAMPLE 48

¹³C NMR (CDCl₃) δ 175.8, 164.7, 162.7, 155.2, 128.6, 128.5, 115.9, 115.8, 115.7, 103.3, 102.3, 86.7, 85.1, 84.7, 84.5, 84.4, 80.1, 80.0, 75.0, 74.4, 74.3, 70.8, 70.9, 69.3, 69.0, 65.5, 65.2, 54.4, 45.4, 40.3, 40.2, 38.8, 38.7, 37.6, 36.8, 36.1, 33.9, 29.7, 28.9, 28.7, 25.6, 24.0, 21.2, 27.0, 20.6, 16.8, 15.6, 15.0, 11.0, 9.1, 8.9.

### EXAMPLE 50

¹³C NMR (CDCl₃) δ 175.9, 175.8, 158.1, 149.6, 149.5, 149.3, 149.2, 136.3, 136.2, 124.1, 124.0, 121.7, 121.6, 103.2, 102.5, 86.7, 84.9, 84.7, 84.3, 84.2, 83.9, 80.8, 80.7, 75.0, 74.4, 71.0, 70.9, 69.2, 68.9, 65.4, 65.1, 54.3, 45.4, 40.3, 38.7, 37.0, 36.3, 36.1, 34.0, 33.9, 29.7, 29.1, 28.8, 28.7, 28.6, 25.6, 25.5, 24.0, 21.2, 20.6, 16.8, 15.5, 15.0, 10.9, 8.9, 8.8.

### EXAMPLE 53

¹³C NMR (CDCl₃) δ 174.3, 157.1, 156.3, 149.1, 136.7, 122.3, 121.8, 103.9, 86.6, 85.0, 84.4, 79.0, 76.7, 74.0, 70.6, 69.3, 65.6, 54.5, 46.1, 44.1, 40.3, 40.2, 37.3, 36.2, 34.4, 29.7, 28.8, 28.6, 25.3, 24.1, 21.1, 20.6, 16.8, 15.4, 14.6, 10.9, 9.4.

### EXAMPLE 55

¹³C NMR (CDCl₃) δ 174.2, 156.1, 150.1, 149.9, 147.8, 124.1, 123.5, 104.2, 86.6, 85.5, 84.4, 78.8, 74.0, 70.6, 69.5, 65.9, 54.5, 44.1, 1.2, 40.3, 37.4, 36.2, 35.6, 34.5, 29.6, 29.2, 28.8, 28.6, 25.4, 24.0, 21.1, 20.5, 16.9, 15.4, 14.7, 10.9, 9.5.

### EXAMPLE 56

¹³C NMR (CDCl₃) δ 176.0, 101.6, 87.0, 84.7, 83.5, 77.3, 75.7, 74.5, 73.6, 68.8, 62.9, 60.2, 45.2, 40.2. 38.1, 36.6, 35.9, 33.8, 33.2, 32.4, 28.5, 25.4, 23.8, 23.7, 20.8, 20.7, 17.2, 15.1, 14.6, 11.0, 10.7, 9.0.

### EXAMPLE 58

¹³C NMR (CDCl₃) δ 176.0, 130.1, 128.0, 126.2, 126.1, 102.5, 86.6, 84.7, 79.9, 79.8, 74.3, 74.0, 70.8, 70.7, 68.9, 65.4, 65.3, 54.3, 45.4, 40.3 38.6, 36.7, 36.0. 33.8, 2937, 29.6, 28.7, 28.6, 25.5, 25.4, 23.9, 21.1. 20.6, 16.8, 16.7, 14.9, 14.8, 10.9, 9.0.

### EXAMPLE 60

¹³C NMR (CDCl₃) δ 174.23, 156.2, 130.2, 129.6, 128.7, 128.6, 126.6, 126.5, 104.2, 86.6, 86.5, 85.7, 85.6, 84.4, 84.3, 79.8, 79.7, 79.0, 76.8, 73.9, 73.8, 70.6, 69.3, 65.56, 5435, 54.4, 44.2, 44.0, 43.9, 40.2, 40.1, 37.8, 37.2, 36.2, 36.1, 34.5, 34.8, 29.7, 29.6, 28.9, 28.6, 25.3, 24.0, 21.1, 21.0, 20.6, 20.5, 17.0, 16.9, 15.4, 14.6, 14.1, 10.9, 9.4, 9.3.

### EXAMPLE 62

¹³C NMR (CDCl₃) δ 174.0, 150.02, 147.2, 122.2, 104.2, 86.6, 85.6, 84.3, 79.5, 74.0, 70.56, 69.41, 65.8, 54.5, 44.1, 40.32, 37.5, 36.1, 28.6, 28.5, 24.0, 21.12 21.1, 20.5, 16.8, 15.5, 14.7, 10.9, 9.6.

### EXAMPLE 64

¹³C NMR (CDCl₃) δ 174.2, 149.0, 148.9, 123.0, 104.2, 86.6, 85.7, 84.3, 74.0, 70.5, 69.4, 65.7, 54.5, 44.1, 42.6, 40.3, 37.3, 36.1, 28.6, 28.5, 25.3, 24.0, 21.1, 20.5, 16.9, 15.5, 14.6, 10.9, 9.5.

### EXAMPLE 66

¹³C NMR (CDCl₃) δ 174.1, 169.6, 154.5, 149.2, 136.4, 124.2, 122.0, 104.3, 86.5, 85.2, 84.3, 79.0, 74.1, 70.7, 69.3, 65.5, 54.4, 43.9, 40.3, 37.5, 36.1, 34.0, 28.6, 25.3, 23.9, 21.1, 20.6, 16.8, 15.5, 14.9, 10.9, 9.4.

### EXAMPLE 68

¹³C NMR (CDCl₃) δ 174.3, 156.1, 150.1, 148.0, 136,3, 123.5, 104.2, 86.6, 85.5, 84.4, 78.8, 74.0, 70.6, 69.5, 35.8, 54.5, 44.1, 41.9, 40.3, 37.4, 36.2, 34.5, 33.5, 29.6, 28.6, 28.8, 25.4, 24.0, 21.1, 20.5, 16.9, 15.4, 14.7, 10.9, 9.5.

### EXAMPLE 70

¹³C NMR (CDCl₃) δ 176.3, 165.1, 149.3, 144.0, 136.3, 116.5, 110.2, 107.2, 86.6, 85.5, 84.4, 78.8, 74.0, 70.6, 69.5, 35.8, 51.5, 44.1, 41.9, 40.3, 37.4, 36.2, 34.5, 33.5, 29.6, 28.6, 27.7, 25.4, 24.0, 20.9, 20.5, 16.9, 15.4, 14.7, 10.9, 9.5.

### EXAMPLE 72

¹³C NMR (CDCl₃) δ 174.2, 163.2, 161.7, 156.3, 130.2, 130.1, 123.1, 114.3, 114.2, 104.2, 86.6, 85.5, 54.4, 79.3, 76.7, 74.0, 70.6, 69.4, 65.7, 54.6, 44.6, 44.1, 41.8, 40.3, 3838, 37.3, 36.2, 34.4, 29.7, 28.6, 27.1, 25.4, 24.1, 21.1, 20.6, 16.9, 15.5, 14.6, 10.9, 9.5.

### EXAMPLE 74

¹³C NMR (CDCl₃) δ 174.2, 161.9, 160.0, 156.1, 130.3, 129.3, 129.2, 125.8, 125.6, 124.2, 115.3, 115.1, 103.8, 86.5, 85.1, 84.3, 79.2, 73.9, 70.4, 69.2, 65.6, 54.4, 44.0, 40.3, 39.2, 37.0, 36.1, 34.2, 29.6, 28.6, 28.5, 25.3, 24.0, 21.1, 20.6, 16.7, 15.5, 14.6, 10.9, 9.4.

### EXAMPLE 76

¹³C NMR (CDCl₃) δ 174.4, 156.5, 136.7, 135.6, 129.0, 127.4, 103.3, 86.3, 84.6, 84.4, 78.6, 76.7, 74.23, 74.1, 70.5, 68.9, 64.8, 54.0, 48.9, 48.8, 48.6, 44.5, 44.4, 43.9, 40.0, 36.9, 35.9, 33.9, 29.0, 28.3, 24.9, 23.98, 20.7, 20.2, 16.6, 15.0, 14.2, 10.7, 9.0.

### EXAMPLE 78

¹³C NMR (CDCl₃) δ 174.2, 171.2, 149.8, 147.9, 135.9, 123.4, 104.6, 86.5, 85.8, 78.6, 74.1, 70.4, 69.6, 65.9, 54.4, 43.7, 40.3, 37.5, 36.2, 35.4, 34.1, 28.6, 28.4, 25.4, 24.0, 21.2, 20.6, 16.8, 15.5, 14.9, 10.9, 9.6, 5.1.

### EXAMPLE 80

¹³C NMR (CDCl₃) δ 174.3, 158.9, 156.1, 130.9, 129.0, 113.9, 103.9, 86.5, 85.4, 84.3, 79.0, 77.4, 74.0, 70.5, 69.2, 65.6, 55.2, 54.4, 44.5, 44.1, 40.3, 37.2, 36.1, 34.3, 28.5, 25.3, 24.0, 21.1, 20.5, 16.8, 15.5, 14.6, 10.9, 9.5.

### EXAMPLE 82

¹³C NMR (CDCl₃) δ 174.2, 156.2, 138.8, 128.6, 127.6, 127.4, 104.0, 86.6, 85.4, 84.4, 79.1, 74.0, 70.6, 69.3, 65.7, 54.5, 45,1, 44.1, 40.3, 37.3, 36.1, 34.4, 28.7, 28.6, 25.4, 24.1, 21.1, 20.6, 16.8, 15.5, 14.7, 10.9, 9.5.

The foregoing is merely illustrative of the invention and is not intended to limit the same to the disclosed compounds and processes. Variations and changes which are obvious to one skilled in the art are intended to be within the scope and nature of the invention as defined in the claims.

## Claims

1. A compound, or a salt, prodrug, or salt of a prodrug thereof, having formula (II) 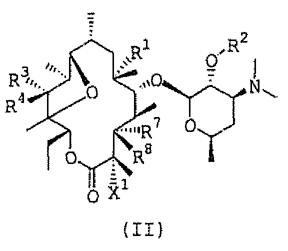 in which
R¹ is hydrogen, -OH, -OR⁹, -OC(O)OR⁹, -OC(O)NH₂, -OC(O)NHR¹⁰, -OC(O)NR¹⁰R¹¹, -OCH₂R¹², -OC(O)OCH₂R¹², -OC(O)NHCH₂R¹², or -OC(O)N(CH₂R¹²)₂;
R² is hydrogen or R^{P}, in which R^{P} is a hydroxyl protecting moiety;
one of R³ and R⁴ is hydrogen, and the other is -OH, -OR¹³, -OC(O)OR¹³, -NH₂, -NHC(O)OR¹⁴, -NHR¹⁵, -NR¹⁵R¹⁶, -OC(O)NH₂, -OC(O)NHR¹⁵, -OC(O)NR¹⁵R¹⁶, -N(R¹⁷)C(O)NH₂, -N(R¹⁷)C(O)NHR¹⁵, -N(R¹⁷)C(O)NR¹⁵R¹⁶, -OCH₂R¹⁸, -NHCH₂R¹⁸, -N(CH₂R¹⁸)₂, -OC(O)OCH₂R¹⁸, -OC(O)NHCH₂R¹⁸, -OC(O)N(CH₂R¹⁸)₂, -N(R¹⁷)C(O)NHCH₂R¹⁸, or -N(R¹⁷)C(O)N(CH₂R¹⁸)₂; or
R³ and R⁴ together are =O or =NOR¹⁹;
R⁷ is hydrogen and R⁸ is -OH, -OR²⁵, -OC(O)R²⁵, -OC(O)OR²⁵, -OC(O)NH₂, -OC(O)NHR²⁶, -OC(O)NR²⁶R²⁷, -OCH₂R²⁸, or -OC(O)OCH₂R²⁸; or
R⁷ and R⁸ together are =O;
R⁹, R¹³, R¹⁹, and R²⁵ are independently alkyl, cycloalkyl, -(CH₂)alkenyl, -(CH₂)alkynyl, alkyl substituted with one substituent selected from the group consisting of cycloalkyl, aryl, heteroaryl, and heterocyclyl, -(CH₂)alkenyl substituted with one substituent selected from the group consisting of cycloalkyl, aryl, heteroaryl, and heterocyclyl, or -(CH₂)alkynyl substituted with one substituent selected from the group consisting of cycloalkyl, aryl, heteroaryl, and heterocyclyl;
R¹⁰, R¹¹, R¹⁵, R¹⁶, R²⁶, and R²⁷ are independently alkyl, cycloalkyl, -(CH₂)alkenyl, -(CH₂)alkynyl, aryl, heteroaryl, heterocyclyl, alkyl substituted with one substituent selected from the group consisting of cycloalkyl, aryl, heteroaryl, heterocyclyl, -NH₂, -NHR³¹, and -NR³¹R³², -(CH₂)alkenyl substituted with one substituent selected from the group consisting of cycloalkyl, aryl, heteroaryl, heterocyclyl, -NH₂, -NHR³¹, and -NR³¹R³², or -(CH₂)alkynyl substituted with one substituent selected from the group consisting of cycloalkyl, aryl, heteroaryl, heterocyclyl, -NH₂, -NHR³¹, and -NR³¹R³²; or
R¹⁰ and R¹¹ together, R¹⁵ and R¹⁶ together,
or R²⁶ and R²⁷ together are independently C₃-C₆-alkylene, C₅-C₆-alkylene interrupted with one moiety selected from the group consisting of -O-, -NH-, -N(alkyl)-, -S-, -S(O)-, and -SO₂-, C₃-C₆-alkylene substituted with one substituent selected from the group consisting of -OH, -O(alkyl), =0, -NH₂, -NH(alkyl), and -N(alkyl)₂, or C₅-C₆-alkylene interrupted with one moiety selected from the group consisting of -O-, -NH-, -N(alkyl)-, -S-, -S(O)-, and -SO₂- and substituted with one substituent selected from the group consisting of -OH, -O(alkyl), =O, -NH₂, -NH(alkyl), and -N(alkyl)₂;
R¹², R¹⁸, and R²⁸ are independently alkyl interrupted with one, two, or three moieties independently selected from the group consisting of -O-, -NH-, -N(alkyl)-, -S-, -S(O)-, and -SO₂- or alkyl interrupted with one, two, or three moieties independently selected from the group consisting of -O-, -NH-, -N(alkyl)-, -S-, -S(O)-, and -SO₂-and substituted with one, two, or three substituents independently selected from the group consisting of cycloalkyl, halo, aryl, heteroaryl, heterocyclyl -OH, =O, -O(alkyl), -NH₂, -NH(alkyl), and -N(alkyl)₂;
R¹⁴ is alkyl or alkyl substituted with one or two independently selected aryl substituents;
R¹⁷ is hydrogen or alkyl;
R³¹ and R³² are independently alkyl, cycloalkyl, aryl, heteroaryl, heterocyclyl, -(CH₂)alkenyl, -(CH₂)alkynyl, alkyl substituted with one substituent selected from the group consisting of cycloalkyl, aryl, heteroaryl, heterocyclyl, -NH₂, -NH(alkyl), and -N(alkyl)₂, -(CH₂)alkenyl substituted with one substituent selected from the group consisting of cycloalkyl, aryl, heteroaryl, heterocyclyl, -NH₂, -NH(alkyl), and -N(alkyl)₂, or -(CH₂)alkynyl substituted with one substituent selected from the group consisting of cycloalkyl, aryl, heteroaryl, heterocyclyl, -NH₂, -NH(alkyl), and -N(alkyl)₂; or
R³¹ and R³² together are C₃-C₆-alkylene, C₅-C₆-alkylene interrupted with one moiety selected from the group consisting of -O-, -NH-, -N(alkyl)-, -S-, -S(O)-, and -SO₂-, C₃-C₆-alkylene substituted with one substituent selected from the group consisting of -OH, -O(alkyl), =O, -NH₂, -NH(alkyl), and -N(alkyl)₂, or C₅-C₆-alkylene interrupted with one moiety selected from the group consisting of -O-, -NH-, -N(alkyl)-, -S-, -S(O)-, and -SO₂- and substituted with one substituent selected from the group consisting of -OH, -O(alkyl), =O, -NH₂, -NH(alkyl), and -N(alkyl)₂; and
X¹ is hydrogen, fluoride, chloride, or bromide.

2. The compound of claim 1, or the salt, prodrug, or salt of the prodrug thereof having the stereochemistry shown in the compound having formula (II)-f 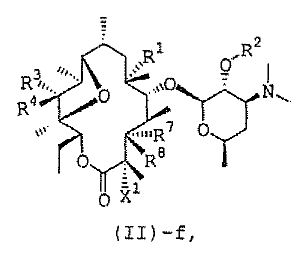 or the compound having formula (II)-g

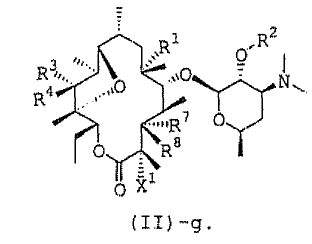

3. The compound of claim 1, or the salt, prodrug, or salt of the prodrug thereof, having formula (II), in which
R¹ is -OH, -OR⁹, -OC(O)OR⁹, -OC(O)NH₂, -OC(O)NHR¹⁰, or -OC(O)NR¹⁰R¹¹;
R² is hydrogen or R^{P}, in which R^{P} is a hydroxyl protecting moiety;
one of R³ and R⁴ is hydrogen, and the other is -OH, -OR¹³, -OC(O)OR¹³, -NH₂, -NHC(O)OR¹⁴, -NHR¹⁵, -NR¹⁵R¹⁶, -OC(O)NH₂, -OC(O)NHR¹⁵, -OC(O)NR¹⁵R¹⁶, -N(R¹⁷)C(O)NH₂, -N(R¹⁷)C(O)NHR¹⁵, or -N(R¹⁷)C(O)NR¹⁵R¹⁶; or
R³ and R⁴ together are =O or =NOR¹⁹;
R⁷ is hydrogen and R⁸ is -OH, -OR²⁵, -OC(O)R²⁵, -OC(O)OR²⁵, -OC(O)NH₂, -OC(O)NHR²⁶, or -OC(O)NR²⁶R²⁷; or
R⁷ and R⁸ together are =O;
R⁹, R¹³, R¹⁹, and R²⁵ are independently alkyl, cycloalkyl, -(CH₂)alkenyl, -(CH₂)alkynyl, alkyl substituted with one substituent selected from the group consisting of cycloalkyl, aryl, heteroaryl, and heterocyclyl, -(CH₂)alkenyl substituted with one substituent selected from the group consisting of cycloalkyl, aryl, heteroaryl, and heterocyclyl, or -(CH₂)alkynyl substituted with one substituent selected from the group consisting of cycloalkyl, aryl, heteroaryl, and heterocyclyl;
R¹⁰, R¹¹, R¹⁵, R¹⁶, R²⁶, and R²⁷ are independently alkyl, cycloalkyl, -(CH₂)alkenyl, -(CH₂)alkynyl, aryl, heteroaryl, heterocyclyl, alkyl substituted with one substituent selected from the group consisting of cycloalkyl, aryl, heteroaryl, heterocyclyl, -NH₂, -NHR³¹, and -NR³¹R³², -(CH₂)alkenyl substituted with one substituent selected from the group consisting of cycloalkyl, aryl, heteroaryl, heterocyclyl, -NH₂, -NHR³¹, and -NR³¹R³², or -(CH₂)alkynyl substituted with one substituent selected from the group consisting of cycloalkyl, aryl, heteroaryl, heterocyclyl, -NH₂, -NHR³¹, and -NR³¹R³²;
R¹⁴ is alkyl or alkyl substituted with one or two independently selected aryl substituents;
R¹⁷ is hydrogen or alkyl;
R³¹ and R³² are independently alkyl, cycloalkyl, aryl, heteroaryl, heterocyclyl, -(CH₂)alkenyl, -(CH₂)alkynyl, alkyl substituted with one substituent selected from the group consisting of cycloalkyl, aryl, heteroaryl, heterocyclyl, -NH₂, -NH(alkyl), and -N(alkyl)₂, -(CH₂)alkenyl substituted with one substituent selected from the group consisting of cycloalkyl, aryl, heteroaryl, heterocyclyl, -NH₂, -NH(alkyl), and -N(alkyl)₂, or -(CH₂)alkynyl substituted with one substituent selected from the group consisting of cycloalkyl, aryl, heteroaryl, heterocyclyl, -NH₂, -NH(alkyl), and -N(alkyl)₂; and
X¹ is hydrogen, fluoride, chloride, or bromide.

4. The compound of claim 3, or the salt, prodrug, or salt of the prodrug thereof, having formula (II), in which
R¹ is -OH or -OR⁹;
R² is hydrogen or R^{p}, in which R^{p} is a hydroxyl protecting moiety;
one of R³ and R⁴ is hydrogen, and the other is -OH, -OR¹³, -OC(O)OR¹³, -NH₂, -NHC(O)OR¹⁴, -NHR¹⁵, -NR¹⁵R¹⁶, -OC(O)NH₂, -OC(O)NHR¹⁵, -OC(O)NR¹⁵R¹⁶, -N(R¹⁷)C(O)NH₂, -N(R¹⁷)C(O)NHR¹⁵, or -N(R¹⁷)C(O)NR¹⁵R¹⁶; or
R³ and R⁴ together are =O;
R⁷ is hydrogen and R⁸ is -OH, -OR²⁵, -OC(O)R²⁵, -OC(O)OR²⁵, -OC(O)NH₂, -OC(O)NHR²⁶, or -OC(O)NR²⁶R²⁷; or
R⁷ and R⁸ together are =O;
R⁹, R¹³, and R²⁵ are independently alkyl, cycloalkyl, -(CH₂)alkenyl, -(CH₂)alkynyl, alkyl substituted with one substituent selected from the group consisting of cycloalkyl, aryl, heteroaryl, and heterocyclyl, -(CH₂)alkenyl substituted with one substituent selected from the group consisting of cycloalkyl, aryl, heteroaryl, and heterocyclyl, or -(CH₂)alkynyl substituted with one substituent selected from the group consisting of cycloalkyl, aryl, heteroaryl, and heterocyclyl;
R¹⁵, R¹⁶, R²⁶, and R²⁷ are independently alkyl, cycloalkyl, -(CH₂)alkenyl, -(CH₂)alkynyl, aryl, heteroaryl, heterocyclyl, alkyl substituted with one substituent selected from the group consisting of cycloalkyl, aryl, heteroaryl, heterocyclyl, -NH₂, -NHR³¹, and -NR³¹R³², -(CH₂)alkenyl substituted with one substituent selected from the group consisting of cycloalkyl, aryl, heteroaryl, heterocyclyl, -NH₂, -NHR³¹, and -NR³¹R³², or -(CH₂)alkynyl substituted with one substituent selected from the group consisting of cycloalkyl, aryl, heteroaryl, heterocyclyl, -NH₂, -NHR³¹, and -NR³¹R³²;
R¹⁴ is alkyl or alkyl substituted with one or two independently selected aryl substituents;
R¹⁷ is hydrogen or alkyl;
R³¹ and R³² are independently alkyl, cycloalkyl, aryl, heteroaryl, heterocyclyl, -(CH₂)alkenyl, -(CH₂)alkynyl, alkyl substituted with one substituent selected from the group consisting of cycloalkyl, aryl, heteroaryl, heterocyclyl, -NH₂, -NH(alkyl), and -N(alkyl)₂, -(CH₂)alkenyl substituted with one substituent selected from the group consisting of cycloalkyl, aryl, heteroaryl, heterocyclyl, -NH₂, -NH(alkyl), and -N(alkyl)₂, or -(CH₂)alkynyl substituted with one substituent selected from the group consisting of cycloalkyl, aryl, heteroaryl, heterocyclyl, -NH₂, -NH(alkyl), and -N(alkyl)₂; and
X¹ is hydrogen, fluoride, chloride, or bromide.

5. The compound of claim 4, or the salt, prodrug, or salt of the prodrug thereof, having formula (II), in which
compounds having
formula (II), formula (II)-f, and formula (II)-g, and pharmaceutically acceptable salts, prodrugs, and salts of prodrugs thereof, in which
R¹ is -OH or -OR⁹;
R² is hydrogen;
one of R³ and R⁴ is hydrogen, and the other is -OH, -OR¹³, NH₂, -NHC(O)OR¹⁴, -NHR¹⁵, -NR¹⁵R¹⁶, -OC(O)NH₂, -OC(O)NHR¹⁵, or -OC(O)NR¹⁵R¹⁶; or
R³ and R⁴ together are =O;
R⁷ is hydrogen and R⁸ is -OH, -OR²⁵, -OC(O)R²⁵, -OC(O)OR²⁵, -OC(O)NH₂, -OC(O)NHR²⁶, or -OC(O)NR²⁶R²⁷; or
R⁷ and R⁸ together are =O;
R⁹, R¹³, and R²⁵ are independently alkyl, -(CH₂)alkenyl, -(CH₂)alkynyl, alkyl substituted with one substituent selected from the group consisting of aryl, heteroaryl, and heterocyclyl, -(CH₂)alkenyl substituted with one substituent selected from the group consisting of aryl, heteroaryl, and heterocyclyl, or -(CH₂)alkynyl substituted with one substituent selected from the group consisting of aryl, heteroaryl, and heterocyclyl;
R¹⁵, R¹⁶, R²⁶, and R²⁷ are independently alkyl, cycloalkyl, -(CH₂)alkenyl, -(CH₂)alkynyl, aryl, heteroaryl, heterocyclyl, alkyl substituted with one substituent selected from the group consisting of aryl, heteroaryl, heterocyclyl, -NH₂, -NHR³¹, and -NR³¹R³², -(CH₂)alkenyl substituted with one substituent selected from the group consisting of aryl, heteroaryl, heterocyclyl, -NH₂, -NHR³¹, and -NR³¹R³², or -(CH₂)alkynyl substituted with one substituent selected from the group consisting of aryl, heteroaryl, heterocyclyl, -NH₂, -NHR³¹, and -NR³¹R³²;
R¹⁴ is alkyl or alkyl substituted with phenyl;
R³¹ and R³² are independently alkyl, -(CH₂)alkenyl, -(CH₂)alkynyl, alkyl substituted with one substituent selected from the group consisting of aryl and heteroaryl, -(CH₂)alkenyl substituted with one substituent selected from the group consisting of aryl and heteroaryl, or -(CH₂)alkynyl substituted with one substituent selected from the group consisting of aryl and heteroaryl; and
X¹ is hydrogen, fluoride, chloride, or bromide.

6. The compound of claim 5, or the salt, prodrug, or salt of the prodrug thereof, having formula (II), in which
compounds having
formula (II), formula (II)-f, and formula (II)-g, and pharmaceutically acceptable salts, prodrugs, and salts of prodrugs thereof, in which
R¹ is -OH or -OR⁹;
R² is hydrogen;
one of R³ and R⁴ is hydrogen, and the other is -OH, -NH₂, -NHR¹⁵, -NR¹⁵R¹⁶ or -NHC(O)OR¹⁴; or
R³ and R⁴ together are =O;
R⁷ is hydrogen and R⁸ is -OH, -OR²⁵, -OC(O)R²⁵, -OC(O)NH₂, -OC(O)NHR²⁶, or -OC(O)NR²⁶R²⁷;
R¹⁵, R¹⁶, R²⁶ and R²⁷ are independently methyl, ethyl, propyl, butyl, prop-2-enyl, prop-2-ynyl, cyclopropyl, cyclopentyl, cyclohexyl, cyclopropyl substituted with phenyl, phenyl substituted with two independently selected halo substituents, or methyl, ethyl, propyl, butyl, prop-2-enyl, or prop-2-ynyl, each of which is substituted with one substitutent selected from the group consisting of (4,5-dihydroisoxazol-5-yl), phenyl, pyridyl, pyrimidinyl, thienyl, isoxazolyl, oxazolyl, quinolyl and isoquinolyl, in which substituent is unsubstituted or substituted with one substituent selected from the group consisting of -F, -Cl, -Br, methyl, -OH, (methyl)O-, phenyl, pyridyl, pyrimidinyl, thienyl and isoxazolyl;
R⁹ and R²⁵ are independently methyl, ethyl, propyl, butyl, prop-2-enyl, or prop-2-ynyl, each of which is independently unsubstituted or substituted with one substituent selected from the group consisting of thienyl, isoxazolyl, 4,5-dihydroisoxazol-5-yl, phenyl, pyridyl, pyrimidinyl, quinolyl, and isoquinolyl, in which each substituent is independently unsubstituted or substituted with one substituent selected from the group consisting of phenyl, pyridyl, pyrimidinyl, thienyl, isoxazolyl, quinolyl, isoquinolyl, and phenyl substituted with one substituent selected from the group consisting of methyl, -OH, (methyl)O-, -F, -Cl, and -Br;
R¹⁴ is tert-butyl or phenylmethyl; and
X¹ is hydrogen, fluoride, chloride, or bromide.

7. The compound of claim 6, or the salt, prodrug, or salt of the prodrug thereof, having formula (II), in which R¹ is -OH, (methyl)O-, (ethyl)O-, (prop-2-ynyl)O-, (prop-2-enyl)O-, (phenylmethyl)O-, (3-(5-pyridin-2-ylthien-2-yl)prop-2-ynyl)O-, (3-(quinolin-3-yl)prop-2-enyl)O-, (3-(3-(pyridin-2-yl)isoxazol-5-yl)prop-2-ynyl)O-, or (3-(5-(pyrimidin-2-yl)thien-2-yl)prop-2-ynyl)O-;
R² is hydrogen;
R³ is hydrogen, and R⁴ is -OH, -NH₂, (tert-butyl)OC(O)NH-, (phenylmethyl)OC(O)NH-, (methyl)NH-, (methyl)₂N-, (ethyl)NH-, (propyl)NH-, (butyl)NH-, (prop-2-ynyl)NH-, (prop-2-enyl)NH-, (methyl)(phenylmethyl)N-, (3-(quinolin-3-yl)prop-2-enyl)NH-, (3-(3-pyridin-2-ylisoxazol-5-yl)prop-2-ynyl)NH-(3-(5-(pyrimidin-2-yl)thien-2-yl)prop-2-ynyl)NH-(3-(quinolin-3-yl)propyl)NH- (3-(quinolin-3-yl)butyl)NH- or (4-(quinolin-3-yl)butyl)NH-; or
R³ and R⁴ together are =O;
R⁷ is hydrogen;
R⁸ is -OH, (methyl)O-, (ethyl)O-, (propyl)O-, (prop-2-ynyl)O-, (prop-2-enyl)O-, (3-(5-(pyridin-2-yl)thien-2-yl)prop-2-ynyl)O-, (3-(quinolin-3-yl)prop-2-enyl)O-, (3-(3-(pyridin-2-yl)isoxazol-5-yl)prop-2-ynyl)O-, (3-(5-(pyrimidin-2-yl)thien-2-yl)prop-2-ynyl)O-, (3-phenyl-4,5-dihydroisoxazol-5-yl)CH₂O-, (3-(pyridin-2-yl)-4,5-dihydroisoxazol-5-yl)CH₂O-, (3-(4-fluorophenyl)-4,5-dihydroisoxazol-5-yl)CH₂O-, ((pyridin-2-yl)methyl)C(O)O-, (2-(pyridin-3-yl)ethyl)C(O)O-, (ethyl)NHC(O)O-, (propyl)NHC(O)O-, (isopropyl)NHC(O)O-, (3,5-dichlorophenyl)NHC(O)O-, (cyclopropyl)NHC(O)O-, (cyclopentyl)NHC(O)O-, (cyclohexyl)NHC(O)O-, (2-phenylcyclopropyl)NHC(O)O-, (phenylmethyl)NHC(O)O-, (2-fluorophenylmethyl)NHC(O)O-, (3-fluorophenylmethyl)NHC(O)O-, (4-fluorophenylmethyl)NHC(O)O-, ((4-methylphenyl)methyl)NHC(O)O-, ((4-methoxyphenyl)methyl)NHC(O)O-, ((pyridin-2-yl)methyl)NHC(O)O-, ((pyridin-3-yl)methyl)NHC(O)O-, ((pyridin-4-yl)methyl)NHC(O)O-, ((3-(phenyl)-4,5-dihydroisoxazol-5-yl)methyl)NHC(O)O-, (2-(pyridin-2-yl)ethyl)NHC(O)O-, (2-(pyridin-3-yl)ethyl)NHC(O)O-, (2-(pyridin-4-yl)ethyl)NHC(O)O-, or (quinolin-4-ylmethyl)NHC(O)O-; and
X¹ is hydrogen, fluoride, chloride, or bromide.

8. The compound of claim 1, or the salt, prodrug, or salt of the prodrug thereof, having formula (II) in which R¹ is -OH; R² is hydrogen; R³ and R⁷ are hydrogen; R⁴ is -NH₂ or -NHC(O)OR¹⁴; R⁸ is -OH, -OR²⁵, -OC(O)R²⁵, or -OC(O)NHR²⁶; R¹⁴ is alkyl substituted with phenyl; R²⁵ is alkyl or alkyl substituted with one substituent selected from the group consisting of pyridyl and 4,5-dihydroisoxazolyl, in which the 4,5-dihydroisoxazolyl is substituted with one substituent selected from the group consisting of pyridyl and phenyl, in which the phenyl is unsubstituted or substituted with one halo substituent; R²⁶ is alkyl, cycloalkyl, cycloalkyl substituted with phenyl, phenyl substituted with two independently selected halo substituents, or alkyl substituted with one substituent selected from the group consisting of phenyl, pyridyl, and 4,5-dihydroisoxazolyl, in which the phenyl is unsubstituted or substituted with one substituent selected from the group consisting of alkyl, halo and -O(alkyl), and the 4,5-dihydroisoxazolyl is substituted with phenyl; and X¹ is hydrogen.

9. The compound of claim 1, or the salt, prodrug, or salt of the prodrug thereof, having formula (II), in which R¹ is -OH; R², R³ and R⁷ are hydrogen; R⁴ is -NH₂ or -NHC(O)OR¹⁴; R⁸ is -OH, -OR²⁵, -OC(O)R²⁵, or -OC(O)NHR²⁶; R¹⁴ is phenylmethyl; R²⁵ is C₃-alkyl or C₁-C₂-alkyl substituted with one substituent selected from the group consisting of pyridyl and 4,5-dihydroisoxazolyl, in which the 4,5-dihydroisoxazolyl is substituted with one substituent selected from the group consisting of pyridyl and phenyl, in which the phenyl is unsubstituted or substituted with one halo substituent; R²⁶ is C₃-alkyl, C₅-C₆-cycloalkyl, C₃-cycloakyl substitued with phenyl, phenyl substitued with two independently selected halo substituents, or C₁-C₂-alkyl substituted with one substituent selected from the group consisting of phenyl, pyridyl, and 4,5-dihydroisoxazolyl, in which the phenyl is unsubstituted or substituted with one substituent selected from the group consisting of methyl, halo and (methyl)O-, and the 4,5-dihydroisoxazolyl is substituted with phenyl; and X¹ is hydrogen.

10. The compound of claim 8, or the salt, prodrug, or salt of the prodrug thereof, having formula (II), in which R¹ is -OH; R², R³ and R⁷ are hydrogen; R⁴ is -NH₂; R⁸ is -OH, (propyl)O-, (3-phenyl-4,5-dihydroisoxazol-5-yl)methoxy, (3-(pyridin-2-yl)-4,5-dihydroisoxazol-5-yl)methoxy, (3-(4-fluorophenyl)-4,5-dihydroisoxazol-5-yl)methoxy, ((pyridin-2-yl)methyl)C(O)O-, (2-(pyridin-3-yl)ethyl)C(O)O-, (propyl)NHC(O)O-, (isopropyl)NHC(O)O-, (cyclopentyl)NHC(O)O-, (cyclohexyl)NHC(O)O-, (2-phenylcyclopropyl)NHC(O)O-, (3,5-dichlorophenyl)NHC(O)O-, (phenylmethyl)NHC(O)O-, ((2-fluorophenyl)methyl)NHC(O)O-, ((3-fluorophenyl)methyl)NHC(O)O-, ((4-fluorophenyl)methyl)NHC(O)O-, ((4-methylphenyl)methyl)NHC(O)O-, ((4-methoxyphenyl)methyl)NHC(O)O-, ((pyridin-2-yl)methyl)NHC(O)O-, ((pyridin-3-yl)methyl)NHC(O)O-, ((pyridin-4-yl)methyl)NHC(O)O-, ((3-(phenyl)-4,5-dihydroisoxazol-5-yl)methyl)NHC(O)O-, (2-(pyridin-2-yl)ethyl)NHC(O)O-, (2-(pyridin-3-yl)ethyl)NHC(O)O-, or (2-(pyridin-4-yl)ethyl)NHC(O)O-; and X¹ is hydrogen.

11. The compound of claim 8, or the salt, prodrug, or salt of the prodrug thereof, having formula (II), in which R¹ is -OH; R², R³ and R⁷ are hydrogen; R⁴ is (phenylmethyl)OC(O)NH-; R⁸ is -OH, (propyl)O-, (3-phenyl-4,5-dihydroisoxazol-5-yl)methoxy, (3-(pyridin-2-yl)-4,5-dihydroisoxazol-5-yl)methoxy, (3-(4-fluorophenyl)-4,5-dihydroisoxazol-5-yl)methoxy, ((pyridin-2-yl)methyl)C(O)O-, (2-(pyridin-3-yl)ethyl)C(O)O-, (propyl)NHC(O)O-, (isopropyl)NHC(O)O-, (cyclopentyl)NHC(O)O-, (cyclohexyl)NHC(O)O-, (2-phenylcyclopropyl)NHC(O)O-, (3,5-dichlorophenyl)NHC(O)O-, (phenylmethyl)NHC(O)O-, ((2-fluorophenyl)methyl)NHC(O)O-, ((3-fluorophenyl)methyl)NHC(O)O-, ((4-fluorophenyl)methyl)NHC(O)O-, ((4-methylphenyl)methyl)NHC(O)O-, ((4-methoxyphenyl)methyl)NHC(O)O-, ((pyridin-2-yl)methyl)NHC(O)O-, ((pyridin-3-yl)methyl)NHC(O)O-, ((pyridin-4-yl)methyl)NHC(O)O-, ((3-(phenyl)-4,5-dihydroisoxazol-5-yl)methyl)NHC(O)O-, (2-(pyridin-2-yl)ethyl)NHC(O)O-, (2-(pyridin-3-yl)ethyl)NHC(O)O-, or (2-(pyridin-4-yl)ethyl)NHC(O)O-; and X¹ is hydrogen.

12. A composition for prophylaxis or treatment of bacterial infections in a fish or a mammal, the composition comprising a therapeutically effective amount of a compound of claim 1.

13. Use of a therapeutically effective amount of a compound of claim 1 for preparation of a medicament for prophylaxis or treatment of bacterial infections.

14. The compound of claim 1, or the salt, prodrug, or salt of the prodrug thereof, which is selected from
(1S,2R,4S,5R,6S,7S,8R,11R,12-(S or R),13S,14R)-13-amino-11-ethyl-4,7-dihydroxy-2,4,6,8,12,14-hexamethyl-9-oxo-10,15-dioxabicyclo(10.2.1)pentadec-5-yl 3,4,6-trideoxy-3-(dimethylamino)-β-D-xylo-hexopyranoside;
benzyl (1S,2R,4S,5R,6S,7S,8R,11R,12-(S or R),13S,14R)-11-ethyl-4, 7-dihydroxy-2,4,6,8,12,14-hexamethyl-9-oxo-5-((3,4,6-trideoxy-3-(dimethylamino)-β-D-xylo-hexopyranosyl)oxy)-10,15-dioxabicyclo(10.2.1)pentadec-13-ylcarbamate;
(1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl isopropylcarbamate;
(1-(S or R),2R,5R,6S,7S,8R,9S,11R,12S,13R,14S)-14-amino-2-ethyl-9-hydroxy-1, 5, 7, 9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl cyclopentylcarbamate;
(1- (S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl cyclohexylcarbamate;
(1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl 4-fluorobenzylcarbamate;
(1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl 3,5-dichlorophenylcarbamate;
(1-(S or R),2R,5R,6S,7S,8R,9S,11R,12S,13R,14S)-14-amino-2-ethyl-9-hydroxy-1,5,7,9,11,13-hexamethyl-4-oxo-8-( (3,4,6-trideoxy-3-(dimethylamino)-β-D-xylo-hexopyranosyl)oxy)-3,15-dioxabicyclo(10.2.1)pentadec-6-yl (1S,2R)-2-phenylcyclopropylcarbamate compound with (1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl (1R,2S)-2-phenylcyclopropylcarbamate (1:1);
(1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl propylcarbamate;
(1S,2R,4S,5R,6S,7S,8R,11R,12-(S or R),13S,14R)-13-amino-11-ethyl-7-((3-(4-fluorophenyl)-4,5-dihydroisoxazol-5-yl)methoxy)-4-hydroxy-2,4,6, 8, 12,14-hexamethyl-9-oxo-10,15-dioxabicyclo(10.2.1)pentadec-5-yl 3,4,6-trideoxy-3-(dimethylamino)-β-D-xylo-hexopyranoside
(1S,2R,4S,5R,6S,7S,8R,11R,12-(S or R),13S,14R)-13-amino-11-ethyl-4-hydroxy-2,4,6,8,12,14-hexamethyl-9-oxo-7-((3-pyridin-2-yl-4,5-dihydroisoxazol-5-yl)methoxy)-10,15-dioxabicyclo(10.2.1)pentadec-5-yl 3,4,6-trideoxy-3-(dimethylamino)-β-D-xylo-hexopyranoside;
(1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl pyridin-2-ylmethylcarbamate;
(1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl 2-pyridin-4-ylethylcarbamate;
(1S,2R,4S,5R,6S,7S,8R,11R,12-(S or R),13S,14R)-13-amino-11-ethyl-4-hydroxy-2,4,6,8,12,14-hexamethyl-9-oxo-7-propoxy-10,15-dioxabicyclo(10.2.1)pentadec-5-yl 3,4,6-trideoxy-3-(dimethylamino)-β-D-xylo-hexopyranoside;
(1S,2R,4S,5R,6S,7S,8R,11R,12-(S or R),13S,14R)-13-amino-11-ethyl-4-hydroxy-2,4,6,8,12,14-hexamethyl-9-oxo-7-((3-phenyl-4,5-dihydroisoxazol-5-yl)methoxy)-10,15-dioxabicyclo(10.2.1)pentadec-5-yl 3,4,6-trideoxy-3-(dimethylamino)-β-D-xylo-hexopyranoside;
(1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl (3-phenyl-4,5-dihydroisoxazol-5-yl)methylcarbamate;
(1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl pyridin-4-ylmethylcarbamate;
(1-(S or R),2R,5R,6S,7S,8R,9S,11R,12S,13R,14S)-14-amino-2-ethyl-9-hydroxy-1,5,7,9, 11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl pyridin-3-ylmethylcarbamate;
(1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl pyridin-2-ylacetate;
(1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl 2-pyridin-3-ylethylcarbamate;
(1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl 2-pyridin-2-ylethylcarbamate;
(1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl 3-fluorobenzylcarbamate;
(1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl 2-fluorobenzylcarbamate;
(1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl 4-methylbenzylcarbamate;
(1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl 3-pyridin-3-ylpropanoate;
(1-(S or R),2R,5R,6S,7S,8R,9S,11R,12S,13R,14S)-14-amino-2-ethyl-9-hydroxy-1,5,7,9,11,13-hexamethyl-4-oxo-8-((3,4,6-trideoxy-3-(dimethylamino)-β-D-xylo-hexopyranosyl)oxy)-3,15-dioxabicyclo(10.2.1)pentadec-6-yl 4-methoxybenzylcarbamate; and
(1-(S or R),2R,5R,6S,7S,8R,9S,11R,12S,13R,14S)-14-amino-2-ethyl-9-hydroxy-1,5,7,9,11,13-hexametbyl-4-oxo-8-((3,4,6-trideoxy-3-(dimethylamino)-β-D-xylo-hexopyranosyl)oxy)-3,15-dioxabicyclo(10.2.1)pentadec-6-yl benzylcarbamate.
